# FASCICULE DE BREVET EUROPEEN

(11) **EP 0 861 837 B1**
(45) Date de publication et mention de la délivrance du brevet: **28.04.2004**
(21) Numéro de dépôt: 98400469.7
(22) Date de dépôt: 27.02.1998
(51) Int. Cl.: C07D 405/04, C07D 409/04, C07D 491/04, C07D 411/04, A61K 31/38, A61K 31/445

(54) **Nouveaux composés de 2-amino indane, leur procédé de préparation et les compositions pharmaceutiques qui les contiennent**
2-Aminoidan Derivate, Verfahren zu deren Herstellung und diese enthaltende pharmazeutische Zusammenstellungen
2-Aminoindane derivatives, process for their preparation and pharmaceutical compositions containing them

(30) Priorité: 27.02.1997 FR 9702360
(43) Date de publication de la demande: 02.09.1998
(73) Titulaire: LES LABORATOIRES SERVIER, 92200 Neuilly sur Seine (FR)
(72) Inventeur: Peglion, Jean-Louis, 78110 Le Vesinet (FR); Goument, Bertrand, 78220 Viroflay (FR); Millan, Mark, 78230 Le Pecq (FR); Gobert, Alain, 93200 Saint-Denis (FR)

(56) Documents cités:
- EP-A- 0 745 598
- WO-A-95/07274

## Description

La présente invention a pour objet de nouveaux composés de 2-amino indane, leur procédé de préparation et les compositions pharmaceutiques qui les contiennent.

Elle concerne plus spécialement les composés de 2-amino indane de formule I : 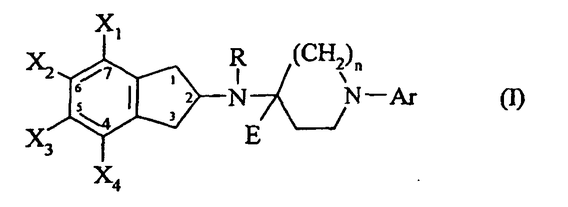 dans laquelle :
- n représente 2 ;
- Ar représente : 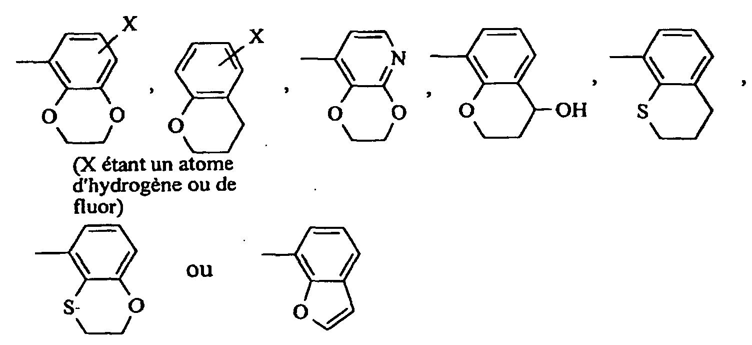
- R représente un atome d'hydrogène,
- E représente un atome d'hydrogène ou un radical méthyle, et
- X₁, X₂, X₃ et X₄, identiques ou différents représentent
   . chacun un atome d'hydrogène ou d'halogène, un radical (C₁-C₅)alkyle ou (C₁-C₅)alkoxy chacun en chaîne droite ou ramifiée, un radical trifluorométhyle, hydroxy, nitro, ou 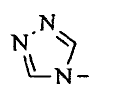
   . et/ou-pris deux-à deux en position adjacente forment, avec les atomes de carbone du noyau phényle auquel ils sont liés un cycle pentagonal composé d'atomes choisis parmi les atomes de carbone, oxygène et azote,
sous forme de mélange racémique et d'isomères optiques quand ils existent,
et leurs sels d'addition acides physiologiquement tolérables.

Les produits de la présente invention peuvent être utilisés comme médicaments dans le traitement de maladies dans lesquelles a été démontré l'implication du système sérotoninergique telles que les maladies psychiatriques (dépression, anxiété, attaque de panique, schizophrénie, agressivité, troubles impulsifs, troubles obsessionnels compulsifs), les maladies dégénératives (Parkinson, Alzheimer), la douleur, la migraine, les céphalées, les accidents vasculaires cérébraux, la boulimie, l'anorexie, l'abus de drogue et aussi dans les maladies cardio-vasculaires (angor instable), puisqu'à l'instar du système nerveux central, le système sérotoninergique est aussi présent dans les territoires cardio-vasculaires. De nombreux récepteurs à la sérotonine ont été identifiés et récemment clonés. Ils ont été classés, sur la base de leur structure primaire et de leur mode de couplage avec les systèmes de transduction, en sept classes majeures 5HT₁ à 5HT₇ (cf. F.G. Boess, Molecular Biology of 5HT Receptors, *Neuropharmacol.,* **1994,** 33, 275). Ces classes sont elles-mêmes subdivisées en sous-types. Pour le récepteur 5HT₁ on connaît les sous-types 5HT_{1A}, 5HT_{1B} (anciennement 5HT_{1Dβ} et 5HT_{1D} (anciennement 5HT_{1Dα}) (pour une revue récente et une discussion de la nomenclature, voir R.P. Hartig, *Trends in Pharmacol*. *Sciences*, **1996**, 17, 103).

L'application de la présente invention concerne plus particulièrement le récepteur 5HT_{1B} car les produits de l'invention se comportent comme des ligands puissants et sélectifs de ce récepteur. Les récepteurs 5HT_{1B} sont localisés post-synaptiquement au niveau cérébral ainsi que sur les terminaisons nerveuses sympathiques périphériques, les vaisseaux sanguins cérébraux, les afférences primaires trigéminales (G.J. Molderings, *Naunyn-Schmiedeberg's Arch. Pharmacol.,* **1990,** 342, 371 ; E. Hamel, *Mol*. *Pharmacol*., **1993,** 44, 242 ; A.T. Bruinvels, *Eur. J Pharmacol*., **1992**, 227, 357). Cette localisation implique que, par activation des populations de récepteurs 5HT_{1B}, par un effet aussi bien vasculaire que neurogénique il est possible de soigner avec des agonistes les migraines et les céphalées. Avec des antagonistes, par action sur les récepteurs périphériques, il sera possible de soigner des maladies du système cardio-vasculaire telles que l'angor instable. D'autre part ces populations de récepteurs 5HT_{1B}, qui sont aussi présentes en fortes concentrations dans la corne dorsale de la moelle épinière, le ganglion basal, l'hippocampe et les autres structures limbiques du cortex frontal (C. Del Arco, *Naunyn-Schmiedeberg's Arch*. *Pharmacol.,* **1992,** 347, 248 ; S. Lowther, *Eur. J*. *Pharmacol*., **1992,** 222, 137 ; X Langlois, *J*. *Neurochem*., **1995,** 65, 2671), peuvent être en partie responsables des troubles de l'humeur et du comportement et être impliqués dans les mécanismes de nociception. Du fait d'une double localisation, d'une part sur les neurones sérotoninergiques post-synaptiques, et d'autre part sur les corps cellulaires où ils jouent le rôle d'autorécepteurs, on peut facilement conclure à leur implication dans la pathogénèse, et par voie de conséquence en utilisant des ligands sélectifs de ces récepteurs, dans les traitement de la dépression, l'anxiété, les troubles impulsifs et les autres maladies psychiatriques liées à un dysfonctionnement de la transmission sérotoninergique (C. Waeber, *Neurochem. Res.,* **1990,** 15, 567; K. Herrick-Davis, *J Neurochem*., **1988,** 51, 1906)

En ce qui concerne le récepteur 5HT_{1B} (ex-5HT_{1Dβ}) il est prédominant dans le système nerveux central de l'homme et du cobaye. De plus, seuls les récepteurs 5HT_{1B} sont localisés comme autorécepteurs, ce qui n'est pas le cas des récepteurs 5HT_{1D} (ex-5HT_{1Dα}). Des ligands des récepteurs 5HT_{1B}/5HT_{1D} ont été décrits dans les demandes WO 96/00720 et WO 96/12713 : il s'agit de dérivés naphthylpipérazine. Des antagonistes des récepteurs 5HT_{1B}/5HT_{1D} de structure biphényliques ont aussi été décrits dans la demande WO 96/19477. Ces structures ne suggèrent en rien les composés de la présente invention.
Des dérivés de pipéridines et pipérazines substituées en position 4 par un hétérobicycle ont été décrits dans la demande EP 0 745 598 et le brevet US 5 464 834. Des dérivés de N-(1,4-benzodioxanyl)-pipérazines ont été décrits dans la demande EP 0 529 462. La demande de brevet WO 95/07274 fait état de composés utilisés dans le traitement des maladies du système nerveux central et possédant une structure 4-amino pipéridine. Dans la formule générale de ces dits composés, l'azote extracyclique est relié, par l'intermédiaire d'une chaîne alkane, à des noyaux benzodioxane, tétrahydronaphtalène et chromane. Ces structures n'induisent donc nullement celles de la présente invention.
L'activité des produits de la présente invention a été démontrée au cours de nombreux tests biologiques et pharmacologiques décrits dans l'étude pharmacologique qui fait l'objet de l'exemple 30 ci-après.
La sélectivité pour les récepteurs 5HT_{1B} a pu être évaluée in vitro au cours d'expérience de binding notamment vis à vis des récepteurs 5HT_{1A}.

Le caractère agoniste ou antagoniste des produits de l'invention a pu être appréhendé grâce au test de l'hypothermie chez le cobaye (M. Stingle et al., *J*. *of Psychopharmacology*, **1994,** 8, 14).
Les expériences de microdialyse montrent l'intérêt des produits de la présente invention dans le traitement des différentes pathologies du système nerveux central. Réalisés dans le cortex frontal ces tests permettent d'envisager, dans le cas où les produits entraînent une augmentation de la libération de sérotonine, leur utilisation dans la dépression, les troubles impulsifs et l'obésité. S'ils produisent une diminution de la libération de sérotonine, ils seront utiles dans l'anxiété, les attaques de panique, les problèmes de sommeil, de cognition et l'abus de drogues. Enfin, s'ils produisent une augmentation de la dopamine et/ou de la noradrénaline, ils seront utiles dans la schizophrénie et comme ci-dessus dans la dépression et les problèmes cognitifs.

La présente invention a également pour objet les compositions pharmaceutiques contenant comme principe actif un composé de formule I ou un de ses sels physiologiquement tolérable mélangé ou associé à un ou plusieurs excipients pharmaceutiques appropriés.

Les compositions pharmaceutiques ainsi obtenues se présentent généralement sous forme dosée renfermant de 0,5 à 25 mg de principe actif. Elles peuvent, par exemple, revêtir la forme de comprimés, dragées, gélules suppositoires, solutions injectables ou buvables et être administrées par la voie orale, rectale ou parentérale selon les formes utilisées.

La posologie varie selon l'âge et le poids du patient, la voie d'administration et les traitements associés et s'échelonne de 0,5 à 25 mg de principe actif, 1 à 3 fois par jour.

La présente invention a également pour objet le procédé de préparation des composés de formule 1 caractérisé en ce que l'on fait réagir :
- soit un composé de formule II : 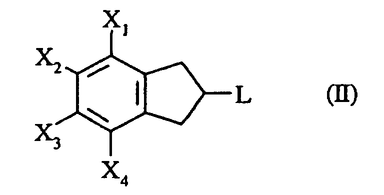 dans laquelle X₁, X₂, X₃ et X₄ ont les significations précédemment définies et L est un groupe labile tel que -OSO₂CH₃, -OSO₂CF₃, 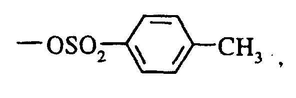 ou un atome d'halogène choisi parmi les atomes de chlore, brome et iode,
   sur un composé de formule III : 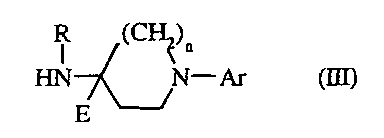 dans laquelle R, E, n et Ar ont les significations précédemment définies,
   cette réaction est plus avantageusement réalisée dans un solvant tel que par exemple la méthylisobutylcétone, en présence de carbonate de métaux alcalins tel que le carbonate de sodium ou de potassium, ou bien dans le toluène en présence de triéthylamine ;
- soit un composé de formule IV : 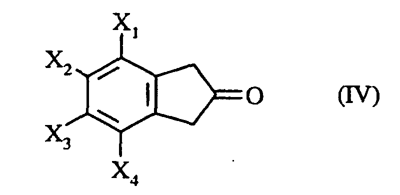 dans laquelle X₁, X₂, X₃ et X₄ ont les significations précédemment définies,
   sur un composé de formule III précédemment définie,
   au cours d'une réaction d'amination réductrice, en présence de borohydrure de sodium dans le tétrahydrofurane ou de triacétoxyborohydrure de sodium dans le dichloroéthane,
- soit encore lorsque E représente uniquement un atome d'hydrogène, un composé de formule V : 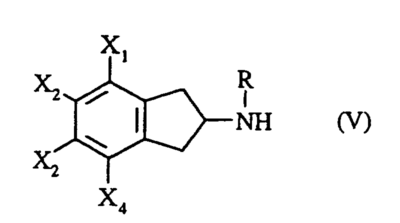 dans laquelle X₁, X₂, X₃, X₄ et R ont les significations précédemment définies,
   sur un composé de formule VI : 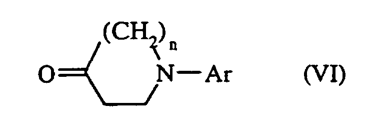 dans laquelle n et Ar ont les significations précédemment définies,
   au cours d'une réaction d'amination réductrice, en présence de borohydrure de sodium dans le tétrahydrofurane ou de triacétoxyborohydrure de sodium dans le dichloroéthane, pour obtenir un composé de formule I' : 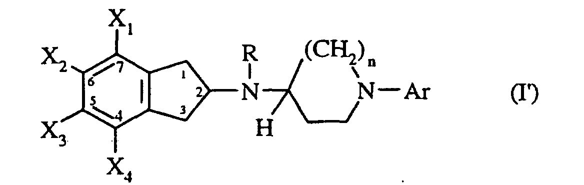 dans laquelle X₁, X₂, X₃, X₄, R, Ar et n ont les significations précédemment définies, (I'étant un sous-ensemble de I)
- soit enfin lorsque le groupement Ar prend la valeur 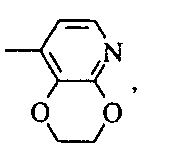 un composé de formule VII : 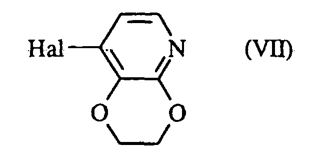 dans laquelle Hal représente un atome d'halogène choisi parmi les atomes de chlore, brome et iode,
   sur un composé de formule VIII : 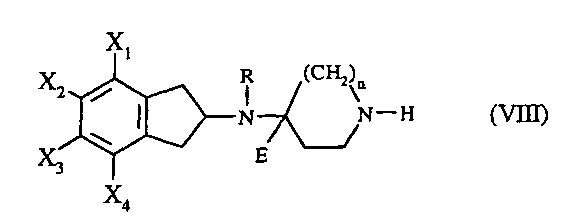 dans laquelle X₁, X₂, X₃, X₄, R, E et n ont les significations précédemment définies,
   en opérant à chaud, dans un solvant tel que par exemple la pyridine, pour obtenir un composé de formule I" : 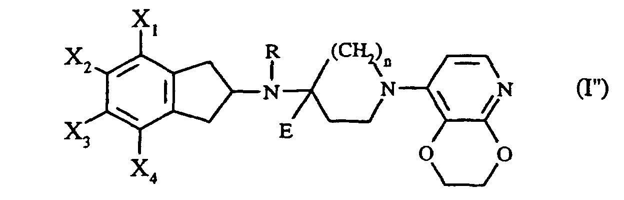 dans laquelle X₁, X₂, X₃, X₄, R, E et n ont les significations précédemment définies,
   (I" étant un autre sous-ensemble de I) ;
   et lorsque l'un ou plusieurs des substituants X₁, X₂, X₃ et X₄ prennent la valeur hydroxy, les composés de formule I précédemment définie et dans laquelle un ou plusieurs des substituants X₁, X₂, X₃ et X₄ représentent un radical hydroxy, peuvent également être préparés à partir des dérivés méthoxy correspondants que l'on traite par le chlorhydrate de pyridine à 200 °C,
en plus, si on le désire, on prépare les isomères optiques des composés de formule I lorsqu'ils renferment un ou plusieurs carbones asymétriques, selon les méthodes classiques de dédoublement connues de la littérature.

Les isomères optiques peuvent aussi être préparés à partir de matières premières optiquement actives.

Les sels des composés de formule I avec des acides pharmaceutiquement acceptables ont été obtenus selon des méthodes classiques comme indiqué dans les exemples ci-après.

Les matières premières sont soit des produits connus soit des produits obtenus à partir de substances connues, selon des procédés connus, comme décrit ci-après dans les préparations 1 à 13.

Les exemples suivants, donnés à titre non limitatif, illustrent la présente invention.

Les points de fusion ont été déterminés soit à la platine chauffante de Kofler (K), soit à la platine chauffante sous microscope (MK).

### Synthèse des matières premières

Les matières premières utilisées dans les exemples suivants ont été préparées comme suit :

### . Préparation 1 : 1-(2,3-dihydro[1,4]benzodioxin-5-yl)-4-amino pipéridine

### Stade 1 : 4-hydroxyimino tétrahydro-4H-pyrane

A température ambiante, on mélange 40,6g (0,406 mole) de tétrahydro-4H-pyran-4-one, 118,7g (1,71 mole) de chlorhydrate d'hydroxylamine et 118,1g (1,44 mole) d'acétate de sodium dans 810 ml d'éthanol, on porte à reflux pendant 20h, puis laisse refroidir. On filtre le solide, le rince à l'éthanol, puis concentre les filtrats. Le résidu est repris par 500 ml d'éther et agité énergiquement pendant 2h (produit insoluble blanchâtre très visqueux). On élimine l'insoluble par filtration et concentre le filtrat pour obtenir 49,5g du produit désiré (théorie : 46,7g) contenant 15% en masse d'acide acétique (Rdt corrigé : 90% environ), et qui est utilisé tel quel.

### Stade 2 : chlorhydrate du 4-amino tétrahydro-4H-pyrane

On mélange 49,3g (= 0,405 mole) du composé précédent et 15 ml de nickel de Raney dans 600 ml d'éthanol, puis on hydrogène le mélange à température ambiante sous 5 .10⁵ Pa d'hydrogène pendant 4h. Après filtration du nickel de Raney, on ajoute 200 ml d'éther chlorhydrique 4,1N (environ 2 éq.), puis évapore les solvants pour recueillir 52,6g du produit désiré (théorie : 55,7g), qui est utilisé tel quel.

### Stade 3 : bromhydrate du 1,5-dibromo-3-amino pentane

A température ambiante, on dissout 52,3g (380 mmol.) du composé précédent dans 380 ml d'acide bromhydrique fumant (à 60%), puis on porte à reflux pendant 24h. On laisse refroidir, puis on ajoute 500 ml d'eau : un solide apparait après quelques minutes. On refroidit dans la glace, puis on filtre ce solide, le rince par très peu d'eau, puis le réempâte dans 200 ml d'éther, le filtre, le rince à l'éther et le sèche sous vide sur potasse. On obtient ainsi 69,5g du produit désiré (Rendement : 56 %) sous forme d'une poudre grise.

### Stade 4 : produit titre

A température ambiante, on mélange 20g (59,0 mmol.) du composé précédent et 8,9g (58,9 mmol.) de 5-amino 1,4-benzodioxane dans 120 ml de chlorobenzène, puis on porte à reflux pendant la nuit. On laisse refroidir : le produit est déposé sur les parois du tricol. On décante la phase chlorobenzène, puis on reprend le résidu par 50 ml d'eau, puis 200 ml d'acide chlorhydrique N. On lave à l'éther (émulsion importante), puis basifie à froid avec de la soude concentrée et extrait 3 fois par 200 ml d'acétate d'éthyle. Les phases organiques jointes sont séchées sur sulfate de magnésium, concentrées (15g), puis chromatographiées sur silice (éluant : dichlorométhane/méthanol/ammoniaque , 95/5/0,5) pour donner 4,7g du produit désiré (théorie : 13,8g) sous forme d'une pâte.

### . Préparation 2 : 1-(2,3-dihydro[1,4]benzodioxin-5-yl)-4-méthylamino pipéridine

### Stade 1 : 1-(2,3-dihydro [1,4]benzodioxin-5-yl)-4-éthoxycarbonylamino pipéridine

A température ambiante, à 1,6g (6,8 mmol.) du composé titre de la préparation 1 dans 20 ml de dichlorométhane, on ajoute d'un trait 2,1 ml (14,9 mmol.) de triéthylamine, puis goutte à goutte en 30 mn 0,71 ml (7,5 mmol.) de chloroformiate d'éthyle. On agite la nuit à température ambiante, puis ajoute 100 ml de dichlorométhane et lave par 100 ml d'eau, 100 ml d'acide chlorhydrique N (2 fois) et 100 ml d'eau. La phase organique est séchée sur sulfate de magnésium et concentrée pour donner 1,3g du produit désiré (Rendement : 65 %).

### Stade 2 : produit titre

A température ambiante, sur 0,32g (8,5 mmol.) d'aluminohydrure de lithium dans 7 ml de tétrahydrofurane, on ajoute goutte à goutte en 15 mn 1,3g (4,2 mmol.) du composé précédent dans 13 ml de tétrahydrofurane. On porte à reflux 1h30, puis laisse la nuit à température ambiante. On hydrolyse à froid successsivement par 0,22 ml d'eau, 0,18 ml de soude à 20% et 0,81 ml d'eau. Après filtration des sels et évaporation du solvant, on obtient 0,77g du produit désiré (Rendement : 73 %).

### . Préparation 3 : 1-(thiochroman-8-yl)-4-amino pipéridine

### Stade 1 : 8-tertiobutyloxycarbonylamino thiochromane

A température ambiante, à 10g (51,5 mmol.) d'acide thiochromane 8-carboxylique dans 260 ml de toluène, on ajoute 9,0 ml (64,4 mmol.) de triéthylamine, puis 13,9 ml (64,4 mmol.) de diphénylphosphoryl azide. On chauffe à 90°C pendant 2h, puis ajoute goutte à goutte 4.8g (64,4 mmol.) de tertiobutanol en solution dans 10 ml de toluène, puis maintient la température à 90° C pendant encore 20h. On laisse refroidir et lave par 120 ml d'eau, 120 ml d'acide chlorhydrique 0,1N, 120 ml d'eau, 120 ml d'une solution aqueuse saturée d'hydrogénocarbonate de sodium et 120 ml d'eau. Après séchage sur sulfate de magnésium et évaporation, le résidu est repris dans le cyclohexane, trituré, et un solide est éliminé par filtration ; le filtrat est concentré (13,3g), puis chromatographié sur 1 kg de silice (éluant : dichlorométhane) pour donner 11,2g du produit désiré (Rendement : 82 %).

### Stade 2 : 8-amino thiochromane

On mélange 10g (37,7 mmol.) du composé obtenu au stade 1 ci-dessus dans 50 ml de dichlorométhane avec 50 ml d'acide trifluoroacétique, agite 30 mn à température ambiante, puis évapore à sec. Le résidu est repris à l'éther, le solide obtenu est filtré et traité par de la soude N. La phase aqueuse est extraite à l'éther, les phases éthérées sont jointes, séchées sur sulfate de magnésium et concentrées pour donner 4,48g du produit désiré (théorie : 6,2g).

### Stade 3 : produit titre

En opérant comme décrit à la préparation 1 stade 4, à partir de 4,6g (27,8 mmol.) du composé obtenu au stade 2 ci-dessus et de 9,4g (27,8 mmol.) de bromhydrate de 1,5-dibromo 3-amino pentane (décrit à la préparation 1 stade 3) dans 60 ml de chlorobenzène, on a obtenu 3,38g du produit désiré (Rendement : 49 %).

### . Préparation 4 : 1-(2,3-dihydro-[1,4]-benzoxathiin-5-yl)pipérid-4-one

### Stade 1 : 3-hydroxy cyclohexylcarboxylate de méthyle

30,4 g (0,2 mol) de 3-hydroxy benzoate de méthyle sont réduits selon la méthode décrite par F. Fache et al. (Tetrahedron Letters, 1995, 36 (6), p 885-888) pour conduire à 14,6 g de produit attendu (Eb/_{133,28 Pa} = 90-95 °C).

### Stade 2 : 3-méthoxycarbonyl cyclohexanone

14,4 g du composé obtenu au stade 1 sont oxydés selon la méthode décrite dans J. Org. Chem., 1965, 30, 145-150 pour conduire à 11,7 g de produit attendu (Eb/_{133.28 Pa} = 80-85 °C), Rendement : 82 %.

### Stade 3 : 3-méthoxycarbonyl cyclohexanone éthylène monothioacétal

11,5 g (73,6 mmol) du produit obtenu au stade 2, 10,3 g de 2-mercapto éthanol, 50 mg d'acide paratoluène sulfonique et 10 ml de toluène sont portés au reflux avec entrainement azéotropique pendant 19 heures. Après évaporation du solvant et de l'excès de 2-mercaptoéthanol, on distille le résidu pour obtenir 6,2 g du produit attendu (Eb/_{66,64 Pa} = 100-110 °C).

### Stade 4 : 5-méthoxycarbonyl-2,3-dihydro[1,4]benzoxathiine

34,1 g (0,16 mol) du produit obtenu au stade précédent sont traités selon la méthode décrite par J.Y. Satah (J.Chem. Soc. Chem. Com., 1985, 1645-6) pour conduire, après deux chromatographies flash sur colonne de silice (éluant CH₂Cl₂), à 1,7 g de produit attendu.

### Stade 5 : Acide 2,3-dihydro[1,4]benzoxathiine-5-carboxylique

1,6 g du composé obtenu au stade 4 sont traités pendant 2 heures à température ambiante par 8 ml de soude 2N et 8 ml de méthanol. Après traitement, on isole 1,3 g de l'acide attendu.

### Stade 6 : N-(2,3-dihydro[1,4]benzoxathiin-5-yl)carbamate de tertiobutyle

Dans un bicol de 100 ml, on mélange 1,25 g du produit obtenu au stade précédent, 1,12 ml de triéthylamine, 1,73 ml de diphénylphosphoryl azide et 32 ml de toluène et porte l'ensemble à 90 °C pendant 3 heures. On ajoute ensuite goutte à goutte en 5 minutes 1,2 ml de tertiobutanol et poursuit le chauffage à 90 °C pendant 20 heures. On laisse refroidir, ajoute 70 ml de toluène et lave 2 fois par 50 ml d'une solution à 10 % de carbonate de sodium puis par 50 ml d'eau. On sèche et évapore pour obtenir 1,7 g de produit attendu.

### Stade 7 : 5-amino-2,3-dihydro[1,4]benzoxathiine

1,5 g du composé obtenu au stade 6 sont dissouts dans l'acétate d'éthyle. On refroidit à 0 °C puis on introduit 5,5 g d'HCl gazeux. On laisse sous agitation pendant 24 heures et filtre sur fritté pour obtenir 0,73 g du chlorhydrate du produit attendu.

### Stade 8 : 1-(2,3-dihydro[1,4]benzoxathiin-5-yl)pipérid-4-ol

0,44 g de la base libre obtenue au stade 7 est mélangée avec 0,41 g de ω,ω'-dichloro pentan-3-ol, 0,20 g d'iodure de sodium, 0,72 g de carbonate de potassium et 1 ml de diméthylformamide et l'ensemble est porté à reflux 2 heures. On dilue par 50 ml d'eau puis extrait à l'éther, lave à la saumure et à l'eau distillée, sèche et évapore. Après purification sur silice, on obtient 0,38 g de produit attendu.

### Stade 9 : produit titre

0,35 g du produit du stade 8, 0,87 g de dicyclohexylcarbodiimide, 0,16 ml de pyridine, 4 ml de diméthylsulfoxyde et 7,5 ml de benzène sont mélangés à 5 °C. On ajoute goutte à goutte 0,1 ml d'acide trifluoroacétique et on agite à température ambiante 21 heures. On dilue à l'acétate d'éthyle, filtre l'insoluble, lave le filtrat à l'eau, le sèche, l'évapore et le purifie sur colonne de silice. On obtient 0,28 g du produit titre attendu.

### . Préparation 5 : 5-([1,2,4]triazol-4-yl)indan-2-yl amine

### Stade 1 : 5-nitro-2-(2-phtalimido)indane

3,1 g (17,2 mmol) de 5-nitro indan-2-yl amine sont mis en suspension dans 23 ml de diméthylformamide. On rajoute 2,75 g (18,6 mmol) d'anhydride phtalique et porte 10 minutes à reflux. Puis, après retour à température ambiante, on coule le milieu réactionnel sur 500 ml d'un mélange eau/glace pour obtenir le produit désiré. (PF (MK) : 195-199 °C).

### Stade 2 : 5-amino-2-(2-phtalimido)indane

2g (6,5 mmol) du composé obtenu au stade précédent sont mis en suspension dans 25 ml de méthanol. On rajoute 100 mg d'oxyde de platine et hydrogène à température ambiante et pression atmosphérique. Après filtration du catalyseur et évaporation du solvant, on obtient 1,7 g du produit attendu (PF(MK) : 294-296 °C), Rendement : 94 %.

### Stade 3 : 5-([1,2,4]triazol-4-yl)-2-(2-phtalimido)indane

0,5 g (1,8 mmol) du produit obtenu au stade 2 et 0,25 g (1,8 mmol) de N,N-diméthylformamide azine sont portés à reflux pendant 17 heures dans 14 ml de toluène en présence de 17 mg d'acide paratoluène sulfonique. On filtre le précipité obtenu, le rince au toluène et le sèche. On obtient 0,22 g du produit attendu (PF (MK) : 224-226 °C), Rendement : 37 %.

### Stade 4 : produit titre

0,22 g (0,6 mmol) du produit obtenu au stade 3 et 43 µl d'hydrate d'hydrazine sont introduits dans 30 ml d'éthanol. On porte 1 heure 30 à reflux. Après addition de 100 ml d'HCl 1N, on filtre le précipité et alcalinise le filtrat par de la soude 1N. On extrait au chlorure de méthylène pour obtenir 0,13 g de produit attendu, Rendement : 100 %.

### . Préparation 6 : 1-(2,3-dihydro[1,4]benzodioxin-5-yl)pyrrolidin-3-one

### Stade 1 : 1-(2,3-dihydro[1,4]-benzodioxin-5-yl)pyrrolidin-3-ol

10 g (66 mmol) de 5-amino-2,3-dihydro [1,4] benzodioxine et 7,65 ml de ω,ω'-dibromo butan-2-ol et 18,25 g de K₂CO₃ dans 125 ml de chlorobenzène sont portés à reflux pendant 18 heures. Après évaporation du solvant, le résidu est repris au chlorure de méthylène et lavé à l'eau. Après séchage et évaporation, on obtient 17,6 g d'une huile qui correspond au produit attendu.

### Stade 2 : produit titre

Sur un mélange composé de 2 g (9,04 mmol) de produit du stade 1, 5,6 g (27 mmol) de N,N-dicyclohexylcarbodiimide, 1,03 ml de pyridine, 25,8 ml de diméthylsulfoxyde et 48 ml de benzène, on coule goutte à goutte 0,51 ml d'acide trifluoroacétique tout en maintenant la température à 5 °C. On laisse ensuite le mélange réactionnel pendant 20 heures sous agitation à température ambiante. On dilue à l'acétate d'éthyle, filtre l'insoluble, lave le filtrat à l'eau, sèche au MgSO₄ et évapore pour donner 2,5 g de produit qui correspond au produit attendu.

### . Préliaration 7 : 1-(chroman-8-yl)-4-amino pipéridine

### Stade 1 : 1-(chroman-8-yl) pipérid-4-one

Obtenue à partir de 8-aminochromane en utilisant les modes opératoires des stades 8 et 9 de la préparation 4.

### Stade 2 : 1-(chroman-8-yl)-4-hydroxyimino pipéridine

Un mélange composé de 1 g (4,32 mmol) du produit obtenu au stade 1, 1,26 g de chlorhydrate d'hydroxylamine, 1,26 g d'acétate de sodium et 20 ml d'éthanol sont portés à reflux pendant 1 heure. Après évaporation du solvant, on reprend par 100 ml de chlorure de méthylène, lave à l'eau, sèche et évapore pour obtenir 0,9 g du produit attendu.

### Stade 3 : Produit titre

0,9 g du produit obtenu au stade 2 est hydrogéné à température ambiante et pression atmosphérique en présence de 1 ml de Ni de Raney et de 1 ml de NH₄OH concentré, dans 20 ml d'éthanol. Après 5 heures de contact, on filtre le catalyseur et évapore à sec. On reprend par 100 ml de chlorure de méthylène et extrait par de l'acide chlorhydrique 1N. La phase acide est basifiée par de la soude concentrée, extraite par du chlorure de méthylène. Après lavage, séchage et évaporation, on isole 0,76 g de produit attendu.

### . Préparation 8 : 1-(2,3-dihydrobenzofuran-7-yl)-4-amino pipéridine

Obtenue comme le produit de la préparation 7 mais en utilisant au stade 1 le 7-amino-2,3-dihydrobenzofurane.

### . Préparation 9 : 1-(benzofuran-7-yl)-4-aminopipéridine

Obtenue comme le produit de la préparation 7 mais en utilisant au stade 1 le 7-amino benzofurane.

### . Préparation 10 : 1-(6-fluorochroman-8-yl)pipérid-4-one

### Stade 1 : 6-fluoro chromane-8-carboxaldéhyde

13,74 g (90,28 mmol) de 6-fluorochromane sont dissouts dans 250 ml de chlorure de méthylène. On refroidit à 0 °C et coule goutte à goutte 20,15 ml de TiCl₄. La solution devient marron et on agite 10 minutes à température ambiante. On introduit alors 8,78 ml (99,3 mmol) d'α,α-dichlorométhyl éther. On agite la nuit à température ambiante. On verse sur de l'eau glacée et décante. La phase organique est séchée et évaporée pour conduire à 17,7 g d'un résidu qui est purifié par chromatographie sur silice (éluant CH₂Cl₂/cyclohexane : 50/50). On obtient alors 6,3 g de produit attendu. Rendement : 38,7 %.

### Stade 2 : Acide 6-fluoro chromane-8-carboxylique

On dissout l'aldéhyde obtenue au stade précédent dans 52 ml d'acétone. On refroidit à 0 °C. On additionne lentement 17,43 ml de réactif de Jones tout en maintenant à une température inférieure à 10 °C. On laisse agiter 4 heures à température ambiante, évapore l'acétone, reprend avec 60 ml d'eau et extrait à l'éther. Les phases éthérées sont extraites avec de la soude 1N. Les phases basiques sont acidifiées à l'acide chlorhydrique concentré et extraites à l'éther. On obtient 5,31 g de produit attendu. Rendement 77,5 %.

### Stade 3 : N-(6-fluoro chromane-8-yl) carbamate de benzyle

On porte 2 heures à 90 °C une solution composée de 137 ml de toluène, 5,3 g (27,07 mmol) d'acide obtenu au stade précédent, 4,72 ml de triéthylamine et 7,29 ml (33,83 mmol) de diphényl phosphorylazide. On ajoute ensuite, tout en maintenant à cette tempérautre, 3,52 ml d'alcool benzylique et laisse 20 heures à la même température. On lave à l'eau, à l'acide chlorhydrique 0,5 N, à nouveau à l'eau puis au bicarbonate de sodium et enfin à l'eau. On sèche et évapore pour obtenir 8,2 g du produit attendu.

### Stade 4 : 8-amino-6-fluoro chromane

8,1 g du composé du stade 3 sont dissouts dans 80 ml d'éthanol. On hydrogène à pression ordinaire et température ambiante en présence de 0,39 g de palladium sur charbon. Après filtration et évaporation, on obtient 4,6 g d'un liquide qui correspond au produit attendu.

### Stade 5 : produit titre

4,5 g du composé obtenu au stade précédent sont traités comme décrits dans les stades 8 et 9 de la préparation 4. On obtient alors 2,9 g du produit attendu.

### . Préparation 11 : 1-(4-acétyloxy chroman-8-yl)-4-amino pipéridine

### Stade 1 : 4-acétyloxy-8-nitro chromane

5,7 g (29,2 mmol) de 4-hydroxy-8-nitro chromane sont dissouts dans 100 ml de chlorure de méthylène. On ajoute 5 ml de triéthylamine puis on coule lentement 2,6 ml de chlorure d'acétyle. On laisse en contact 1 heure 30 puis on ajoute 2 ml de triéthylamine et coule lentement 1 ml de chlorure d'acétyle. On laisse encore 1 heure à température ambiante, dilue au chlorure de méthylène, lave à l'eau, à l'acide chlorhydrique 1N, puis avec une solution à 5 % de carbonate de sodium et enfin avec 100 ml d'eau. On sèche, évapore pour obtenir 6,6 g d'une huile orange qui correspond au produit attendu. Rendement : 95 %.

### Stade 2 : 4-acétyloxy-8-amino chromane

On dissout 6,6 g (27,8 mmol) du produit du stade 1 dans 85 ml de méthanol. On rajoute 0,43 g d'oxyde de platine et hydrogène à température ambiante et pression ordinaire pendant 4 heures. On filtre le catalyseur, le rince et évapore le filtrat pour obtenir 5,5 g d'une huile visqueuse qui correspond au produit attendu. Rendement : 96 %.

### Stade 3 : 1-(4-acétyloxy chromane)pipérid-4-one

7,5 g (30,7 mmol) de la base obtenue au stade 2 sont traités selon les modes opératoires décrits dans les stades 8 et 9 de la préparation 4. On obient alors 0,4 g du produit attendu.

### Stade 4 : 1-(4-acétyloxy chroman-8-yl)-4-hydroxyimino pipéridine

0,4 g du composé obtenu au stade précédent est traité selon le mode opératoire décrit au stade 2 de la préparation 7 pour conduire à 0,28 g de produit attendu. Rendement : 66 %.

### Stade 5 : produit titre

0,28 g du composé du stade 4 est mis en solution dans une solution de 6 ml d'éthanol et 0,3 ml de NH₄OH. On rajoute 0,3 ml de Nickel de Raney et hydrogène à température ambiante sous pression atmosphérique. Après filtration du catalyseur, rinçage et évaporation, on obtient 0,25 g de produit attentu. Rendement : 96 %.

### . Préparation 12 : 4-amino-1-(2,3-dihydro [1,4]benzodioxin-5-yl)-4-méthyl pipéridine

### Stade 1 : 1-(2,3-dihydro [1,4] benzodioxin-5-yl)-4-hydroxy-4-méthyl pipéridine

On dissout 3,5 g (15 mmol) de 1-(2,3-dihydro [1,4] benzodioxin-5-yl) pipérid-4-one dans 30 ml d'éther. On refroidit à 0 °C et on ajoute, en maintenant à cette température, 10 ml d'une solution 3 M de bromure de méthylmagnésium dans l'éther. On laisse agiter 1 heure à cette température puis ½ heure à température ambiante. On hydrolyse en versant sur 100 ml d'une solution aqueuse saturée de NH₄Cl. On extrait par 100 ml d'éther. On sèche et évapore pour obtenir un résidu de 3,3 g que l'on purifie par chromatographie flash sur silice (éluant CH₂Cl₂/CH₃COOC₂H₅ : 90/10). On isole ainsi 1,3 g de produit attendu.

### Stade 2 : 4-acétylamino-1-(2,3-dihydro [1,4] benzodioxin-5-yl)-4-méthyl pipéridine

1,03 g (4,1 mmol) du produit du stade 1 sont dissouts dans 4 ml d'acétonitrile. On ajoute goutte à goutte en 5 minutes 1 ml d'H₂SO₄ concentré en maintenant à une température inférieure à 40 °C. On agite la nuit à température ambiante, puis verse sur 50 ml de soude 1N glacée. On extrait par du chlorure de méthylène, lave à l'eau, sèche et évapore pour obtenir 0,85 g d'un résidu que l'on purifie par chromatographie flash sur silice (eluants : chlorure de méthylène/acétate d'éthyle : 50/50, puis chlorure de méthylène/méthanol 98/2). On obtient 0.54 g de produit attendu.

### Stade 3 : produit titre

0,51 g (1,76 mmol) du produit obtenu au stade 2 est traité à reflux une nuit par 3 ml d'HCl 3N. On rajoute 1 ml d'HCl concentré et on porte encore 24 heures à reflux. On laisse refroidir, basifie par NaOH 2N et extrait 3 fois par 30 ml de CH₂Cl₂. Les phases organiques sont lavées puis séchées. On obtient 0,32 g de produit attendu. Rendement : 73 %.

### . Préparation 13 : 6-amino cyclopenta [f] [2,1,3]benzoxadiazole et son chlorhydrate

### Stade 1 : 2-acétylamino-5-amino-6-nitro indane

Sur 2,85 g (15 mmol) de 2-acétylamino-5-amino indane dans 7,5 ml d'H₂SO₄ concentré refroidis à 0 °C, on introduit par fractions 1,62 g de KNO₃. On laisse sous agitation 3 heures à 0 °C puis verse sur de la soude 6 N glacée, on agite, extrait au chlorure de méthylène puis lave, sèche et évapore. On isole 2,8 g d'un solide noir qui est purifié sur silice (éluant : chlorure de méthylène/méthanol : 97/3). On isole ainsi 1,46 g de produit attendu. Rendement : 42 %.

### Stade 2 : 6-acétylamino cyclo penta [f] [2,1,3] benzoxadiazole

Sur 3 ml d'acide sulfurique à 0 °C, on ajoute 0,46 g (6,6 mmol) de NaNO₂, puis 1,4 g (6 mmol) du produit du stade 1 en solution dans 10 ml d'acide acétique. Après 15 minutes à 0°C, on verse dans 20 g de glace. La solution ainsi obtenue est versée goutte à goutte sur une solution vigoureusement agitée de 600 mg (9,2 mmol) d'azide de sodium dans 12 ml d'eau. On agite 10 minutes à température ambiante et extrait 3 fois par 40 ml de CH₂Cl₂. Les phases organiques sont lavées par une solution aqueuse à 10 % de carbonate de sodium. On sèche sur MgSO₄, filtre et rajoute 100 ml de toluène sec. On évapore le chlorure de méthylène et porte à reflux la solution toluénique ainsi obtenue pendant 3 heures. On laisse refroidir, filtre le solide, le rince au toluène et le sèche sous vide. Ce solide est alors dissout dans 50 ml d'éthanol et on rajoute 5 ml de triéthylphosphite. On porte à reflux pendant 1 heure 30 minutes. On évapore pour isoler 0,91 g de produit attendu.

### Stade 3 : produit titre

0,87 g du produit obtenu au stade 2 est dissout dans 10 ml de méthanol. On rajoute 5 ml d'HCl 6N et porte à reflux 24 heures. Après évaporation, on isole 0,88 g de chlohrydrade du produit attendu.

### Exemple 1:

### 1-(2,3-dihydro[1,4] benzodioxin-5-yl)-4-[N-(indan-2-yl) amino] pipéridine et son dichlorhydrate

On porte à reflux, pendant 24heures, un mélange de 11 g (38 mmol) du tosylate de l'indan-2-ol, 9g (38 mmol.) du composé titre de la préparation 1, 7,7g (76 mmol.) de triéthylamine et 150 ml de toluène. On évapore à sec, reprend le résidu par du dichlorométhane et de la soude 1N, décante, lave la phase organique à l'eau, sèche sur MgSO₄ et concentre. Le résidu est purifié par chromatographie sur silice (éluant : CH₂Cl₂ / CH₃COOC₂H₅ , 90/10). Le produit obtenu qui correspond à la structure attendue est transformé en dichlorhydrate par action de l'éther chlorhydrique. Rendement = 15%. P.F. > 260° C.

### Exemple 3 :

### 1-(2,3-dihydro[1,4] benzodioxin-5-yl)-4-[N-(5,6-méthylènedioxy indan-2-yl) amino] pipéridine et son fumarate

On agite à température ambiante pendant une nuit 1,0g (4,3 mmol.) de 1-(2,3-dihydro [1,4] benzodioxin-5-yl) 4-amino pipéridine (décrit à la préparation 1), 0,75g (4,3 mmol.) de 5,6-methylènedioxy indan-2-one et 2g de tamis moléculaire 4Å dans 16 ml de chloroforme. On filtre alors le tamis, le rince par un peu de chloroforme et concentre le filtrat. Le résidu est repris par 16 ml de méthanol et 4 ml de tétrahydrofurane, puis on ajoute d'un trait 0,32g (8,5 mmol.) de borohydrure de sodium ; on laisse agiter la nuit à température ambiante, puis évapore à sec. Le résidu est repris par 100 ml de dichlorométhane et lavé 2 fois par 50 ml d'eau. Après séchage sur sulfate de magnésium et concentration, le résidu (1,66g) est chromatographié sur 160g de silice (éluant : dichlorométhane/méthanol, 98/2) pour donner 0,71g du produit attendu sous forme de base libre.

Le fumarate correspondant est obtenu dans l'éthanol, par addition d'un équivalent d'une solution d'acide fumarique à 2% dans l'éthanol. Après filtration et séchage, on obtient 0,68g du fumarate attendu dont le PF (M.K.) est de 255-259° C. Rendement = 31%.

### Exemple 4 :

### 4-[N-(indan-2-yl) amino]-1-(thiochroman-8-yl) pipéridine

Préparé de la même façon que le composé de l'exemple 3 mais en utilisant le composé de la préparation 3 à la place de la 4-amino-1-(2,3-dihydro[1,4] benzodioxin-5-yl) pipéridine et l'indan-2-one à la place de la 5,6-méthylènedioxy indan-2-one. Après purification sur silice (éluant : CH₂Cl₂/CH₃OH, 98/2), on obtient le produit attendu qui fond (M.K.) à 109-112°C. Rendement = 8%.

### Exemple 5 :

### 1-(2.3-dihydro[1,4] benzodioxin-5-yl)-4-[N-(5,6-diméthoxy indan-2-yl) amino] pipéridine et son fumarate

Préparé de la même façon que le composé de l'exemple 3 mais en utilisant la 5,6-diméthoxy indan-2-one à la place de la 5,6-méthylènedioxy indan-2-one. Après purification sur silice en utilisant le même éluant, on obtient le produit attendu que l'on transforme en fumarate correspondant dont le point de fusion (M.K.) est de 212-216°C. Rendement = 12%

### Exemple 6 :

### 1-(2,3-dihydro[1,4] benzodioxin-5-yl)-4-[N-(5-méthyl indan-2-yl) amino] pipéridine et son fumarate

Préparé comme le composé de l'exemple 3 mais en utilisant la 5-méthyl indan-2-one à la place de la 5,6-méthylènedioxy indan-2-one. Après purification en utilisant les mêmes conditions, on obtient le produit attendu dont le fumarate correspondant fond (M.K.) à 239-241 ° C. Rendement = 19%.

### Exemple 7 :

### 1-(2,3-dihydro[1,4] benzodioxin-5-yl)-4-[N-(5-chloro indan-2-yl) amino] pipéridine et son fumarate

Préparé comme le composé de l'exemple 3 mais en remplaçant la 5,6-méthylène dioxy indan-2-one par la 5-chloro indan-2-one. Après purification sur silice en utilisant le même éluant, on obtient le produit attendu dont le fumarate correspondant fond (M.K.) à 238-241°C. Rendement = 9%.

### Exemple 8 :

### 1-(2,3-dihydro [1,4] benzodioxin-5-yl)-4-[N-(5-méthoxy indan-2-yl)amino] pipéridine et son fumarate

Préparé comme le composé de l'exemple 3, mais en utilisant la 5-méthoxy indan-2-one à la place de la 5,6-méthylènedioxy indan-2-one. Le fumarate du produit attendu fond (MK) à 222-227 °C.

### Exemple 9 :

### 1-(2,3-dihydro [1,4] benzodioxin-5-yl)-4-[N-(5,6-diméthyl indan-2-yl) amino] pipéridine et son fumarate

Préparé comme le composé de l'exemple 3, mais en utilisant la 5,6-diméthyl indan-2-one à la place de la 5,6-méthylène dioxy indan-2-one. Le fumarate de produit attendu fond (MK) à 232-235 °C.

### Exemple 10 :

### 1-(2,3-dihydro[1,4]benzodioxin-5-yl)-4-[N-(5-trifluorométhyl indan-2-yl) amino] pipéridine et son fumarate

Préparé comme le composé de l'exemple 3, mais en utilisant la 5-trifluorométhyl indan-2-one à la place de la 5,6-méthylènedioxy indan-2-one. Le fumarate du produit attendu fond (MK) à 228-232 °C.

### Exemple 11 :

### 1-(2,3-dihydro [1,4] benzodioxin-5-yl)-4-[N-(4,7-diméthoxy indan-2-yl) amino]pipéridine et son hémifumarate

Préparé comme le composé de l'exemple 3, mais en utilisant la 4,7-diméthoxy indan-2-one à la place de la 5,6-méthylènedioxyindan-2-one. L'hémifumarate du produit attendu fond (MK) à 217-220 °C.

### Exemple 12 :

### 1-(2,3-dihydro [1,4] benzodioxin-5-yl)-4-[N-(5-fluoroindan-2-yl)amino] pipéridine et son fumarate

Préparé comme le composé de l'exemple 3, mais en utilisant la 5-fluoroindan-2-one à la place de la 5,6-méthylènedioxy indan-2-one. Le fumarate du produit attendu fond (MK) à 216-220 °C.

### Exemple 14 :

### 1-(2,3-dihydro [1,4] benzoxathiin-5-yl)-4-(indan-2-yl amino) pipéridine et son hémifumarate

0,17 g (1 mmol) de chlorhydrate d'indan-2 yl amine est introduit dans 7 ml de 1,2-dichloroéthane puis on ajoute dans l'ordre : 0,14 ml (1,5 mmol) de triéthylamine, 0,25 g de 1-(2,3-dihydro [1,4] benzoxathiin-5-yl)pipérid-4-one (préparation 4), 0,32 g (1,5 mmol) de triacétoxyborohydrure de sodium et 58 µl (1 mmol) d'acide acétique. On laisse 20 heures sous agitation à température ambiante puis verse dans 10 ml de soude 1N et extrait 2 fois par 25 ml d'éther. Les phases organiques jointes sont lavées et séchées. On obtient après évaporation 0,36 g du produit titre que l'on transforme en hémifumarate par action d'une solution d'acide fumarique à 2 % dans l'éthanol. On obtient ainsi 0,27 g d'hémifumarate. PF (MK) = 220-223 °C.

### Exemple 15 :

### 1-(chroman-8-yl)-4-(indan-2-yl amino)pipéridine et son fumarate

Obtenu comme le produit de l'exemple 14 mais en utilisant la 1-(chroman-8-yl)pipérid-4-one à la place du produit de la préparation 4. Le fumarate correspondant fond (MK) à 236-240 °C.

### Exemple 17 :

### 1-(6-fluoro chroman-8-yl)-4-(indan-2-yl amino) pipéridine et son fumarate

Obtenu de la même façon que le produit de l'exemple 14, mais en utilisant la 1-(6-fluorochroman-8-yl) pipérid-4-one (préparation 10) à la place du produit de la préparation 4. Le fumarate du produit attendu fond (MK)à 226-230 °C.

### Exemple 18 :

### 1-(2,3-dihydro [1,4] benzodioxin-5-yl)-4-(5-nitro indan-2-yl amino) pipéridine et son fumarate

Obtenu de la même façon que le produit de l'exemple 14 mais en utilisant le chlorhydrate de 5-nitro indan-2-ylamine à la place du chlorhydrate d'indan-2-yl amine et la 1-(chroman-8-yl) pipérid-4-one à la place du produit de la préparation 4. Le fumarate du produit titre fond (MK) à 234-237 °C.

### Exemple 19 :

### 1-(chroman-8-yl)-4-[5-(1,2,4-triazol-4-yl) indan-2-yl amino] pipéridine et son hémifumarate

Obtenu de la même façon que le produit de l'exemple 14 mais en utilisant le chlorhydrate de 5-(1,2,4-triazol-4-yl) indan-2-yl amine (préparation 5) à la place du chlorhydrate d'indan-2-yl amine et la 1-(chroman-8-yl) pipérid-4-one à la place du produit de la préparation 4. L'hémifumarate fond (MK) à 153-155 °C.

### Exemple 20 :

### 1-(chroman-8-yl)-4-[(cyclopenta [f] [2,1,3] benzoxadiazol-6-yl) amino] pipéridine et son fumarate

Obtenu de la même façon que le produit de l'exemple 14 mais en utilisant le chlorhydrate de 6-amino cyclopenta [f] [2,1,3] benzoxadiazole (préparation 13) à la place du chlrohydrate d'indan-2-yl amine et la 1-(chroman-8-yl) pipérid-4-one à la place du produit de la préparation 4. Le fumarate du produit titre fond (MK) à 225-227 °C.

### Exemple 21 :

### 1-(chroman-8-yl)-4-[(5-fluoro indan-2-yl) amino] pipéridine et son fumarate

On mélange 0,49 g (3,27 mmol) de 5-fluoro indan-2-one et 0,76 g (3,27 mmol) de 1-(chroman-8-yl)-4-amino pipéridine (préparation 7) dans 25 ml de 1,2-dichloroéthane. On introduit ensuite 1,1 g de triacétoxyborohydrure de sodium et 0,19 ml d'acide acétique. On agite pendant 24 heures. Le milieu réactionnel est versé sur de la soude 1N et extrait à l'éther. Les phases organiques sont lavées, séchées et évaporées. Le résidu est purifié sur colonne de silice (éluant CH₂Cl₂/C₂H₅OH : 98/2).
On obtient 0,64 g d'un liquide dont la structure correspondant au produit attendu et qui est traité par un solution éthanolique d'acide fumarique à 2 %. On obtient ainsi 0,67 g du fumarate du produit attendu. PF (MK) : 227-231 °C.

### Exemple 22 :

### 1-(2,3-dihydrobenzo furan-7-yl)-4-[(5-fluoro indan-2-yl)amino] pipéiridine et son hémifumarate

Obtenu de la même façon que le produit de l'exemple 21 mais en utilisant la 1-(2,3-dihydrobenzofuran-7-yl)-4-amino pipéridine (préparation 8) à la place du produit de la préparation 7. L'hémifumarate du produit attendu fond (MK) à 225-229 °C.

### Exemple 23 :

### 1-(chroman-8-yl)-4-[(5,6-méthylènedioxyindan-2-yl) amino] pipéridine et son fumarate

Obtenu de la même façon que le produit de l'exemple 21 mais en utilisant la 5,6-méthylènedioxy indan-2-one à la place de la 5-fluoroindan-2-one. Le fumarate du produit attendu fond (MK) à 248-252 °C.

### Exemple 24 :

### 1-(benzofuran-7-yl)-4-[(5-fluoro indan-2-yl)amino] pipéridine et son hémifumarate

Obtenu de la même façon que le produit de l'exemple 21 mais en utilisant la 1-(benzofuran-7-yl)-4-aminopipéridine (préparation 9) à la place du produit de la préparation 7. L'hémifumarate du produit attendu fond (MK) à 219-222 °C.

### Exemple 25 :

### 1-(chroman-8-yl)-4-[(5-méthoxyindan-2-yl)amino]pipéridine et son fumarate

Obtenu de la même façon que le produit de l'exemple 21 mais en utilisant la 5-méthoxy indan-2-one à la place de la 5-fluoro indan-2-one. Le fumarate du produit attendu fond (MK) à 218-223 °C.

### Exemple 26 :

### 1-(4-hydroxy chroman-8-yl)-4-[(5,6-méthylènedioxy indan-2-yl)amino] pipéridine et son hémifumarate

### Stade 1 : 1-(4-acétyloxy chroman-8-yl)-4-[(5,6-méthylènedioxy indan-2-yl)amino]pipéridine

Obtenu de la même façon que le produit de l'exemple 21 mais en utilisant la 5,6-méthylènedioxy indane-2-one à la place de la 5-fluoro indan-2-one et la 1-(4-acétyloxychroman-8-yl)-4-amino pipéridine (préparation 11) à la place du produit de la préparation 7. On isole une huile jaune qui correspond au produit attendu avec un rendement de 26 %.

### Stade 2 : Produit titre

100 mg (0,22 mmol) du produit obtenu au stade 1 sont dissouts dans une solution de 2 ml de méthanol et 1,5 ml de soude 1N. On laisse en contact une nuit, puis 1 heure à 60 °C. Après retour à température ambiante, on évapore le méthanol, dilue à l'eau et extrait au chlorure de méthylène. Après séchage et évaporation, on isole 60 mg d'un produit qui correspond à la structure attendue que l'on transforme en fumarate par action d'une solution à 2 % d'acide fumarique dans l'éthanol. On isole 0,44 g d'un produit qui correspond à l'hémifumarate du produit attendu. PF(MK) : 252-253 °C.

### Exemple 27 :

### 1-(2,3-dihydro[1,4] benzodioxin-5-yl)-4-(indan-2-yl amino)-4-méthyl pipéridine et son fumarate

Obtenu de la même façon que le composé de l'exemple 21 mais en utilisant l'indan-2-one à la place de la 5-fluoro indan-2-one et la 4-amino-1-(2,3-dihydro[1,4]-benzodioxin5-yl)-4-méthylpipéridne (préparation 12) à la place du produit de la préparation 7. Le fumarate du produit attendu fond (MK) à 218-223 °C.

### Exemple 28 :

### 1-(2,3-dihydro[1,4]-benzodioxin-5-yl)-4-[N-(5-hydroxy indan-2-yl)amino]pipéridine et son hémifumarate

1 g (2,2 mmol) du dichlorhydrate du produit de l'exemple 8 et 0,77 g (6,6 mmol) de chlorhydrate de pyridine sont mélangés et portés à 200 °C pendant 1 heure. On laisse revenir à température ambiante et reprend par 100 ml de chlorure de méthylène et 100 ml de solution diluée d'ammoniaque. On sépare les phases, sèche la phase organique et évapore le solvant. On obtient 0,78 g du produit attendu que l'on transforme en 0,61 g d'hémifumarate correspondant. PF(MK) : 250-260°C.

### Exemple 29 :

### 1-(chroman-8-yl)-4-[N-(5-hydroxy indan-2-yl)amino]pipéridine et son hémifumarate

On opére de la même façon qu'à l'exemple 28 mais en utilisant le produit de l'exemple 25 à la place du produit de l'exemple 8. L'hémifumarate du produit attendu fond (MK) au-dessus de 350 °C.

### Exemple 30

### ETUDE PHARMACOLOGIOUE

### ◆ Etudes de liaison

### . Liaison 5-HT_{1B}

### a) Préparation des membranes

Après dissection des cerveaux de cobaye, les striatums extraits sont congelés puis homogénéisés dans 20 volumes (poids/volume) de 50mM Tris-HCl (pH 7.7 à température ambiante) contenant 4mM de CaCl₂, 0,1% d'acide ascorbique, et centrifugées à 48.000g pendant 25 minutes à 4° C. Le surnageant est séparé et le précipité est remis en suspension dans le même volume de tampon avant d'être incubé à 37° C pendant 15 minutes pour retirer la sérotonine endogène. Finalement la suspension est centrifugée à 48.000g pendant 25 minutes à 4° C et le précipité remis en suspension dans 80 volumes de tampon qui contient 10 µM de pargylline.

### b) Etude de liaison

Les études de liaison ([³H]-GR 125743) sont effectuées en triple dans le tampon suivant : 50 mM Tris-HCl (pH 7,7 à température ambiante) contenant 4mM de CaCl₂, 0,1 % d'acide ascorbique et 10 µM de pargylline. Le volume final de 500 µl est constitué de 100 µl de radioligand, 100 µl de tampon ou de composé à tester et 300 µl de membranes. La sérotonine (10 µM) est utilisée pour définir la liaison non spécifique. Dans les expériences de compétition, la concentration de [³H]-GR 125743 est de 1 nM. Les incubations sont initiées par l'addition de la préparation membranaire et durent 60 minutes à température ambiante. La réaction est stoppée par filtration rapide au travers de filtres Whatman GF/B pré-traités avec 0.1% de polyéthylènimine, suivi par trois rinçages avec du tampon froid. La liaison spécifique représente environ 90% de la liaison totale aux concentrations de radioligand proche de la valeur du Kd.

### . Analyses de données

Les données sont analysées par régression non linéaire en utilisant le programme P_{RISM} (Graphpad Software Inc., San Diego, CA) pour déterminer les valeurs du Kd (constante de dissociation du radioligand), du Bmax (nombre de sites maximum) pour les expériences de saturation et celles de l'IC₅₀ (concentration inhibitrice 50) et du nombre de Hill pour les expériences de compétition. La constante d'inhibition (Kᵢ) est calculée selon l'équation de Cheng-Prussof : Kᵢ = IC₅₀/1+L_{/Kd} dans laquelle L représente la concentration du radioligand.
Les résultats sont exprimés en pKᵢ = - log Kᵢ.
Les composés de la présente invention montrent une très bonne affinité pour le récepteur 5HT_{1B}. A titre d'exemple le pKᵢ du composé de l'exemple 3 est de 8,5.

### . Liaison 5HT_{1A}

Les études de liaison sur le récepteur 5HT_{1A} ont été effectuées selon les méthodes connues et décrites dans la littérature (cf. S.J. Peroutka, *J. Neurochem*., **1986**, 47, 529-40, M.J. Millan, *J. Pharmacol*. *Exp. Ther.,* **1994**, 268, 337-52). Les résultats sont aussi exprimés en pKᵢ. Les composés de la présente invention sont très peu affins pour le récepteur 5HT_{1A}. A titre d'exemple le pKᵢ du composé de l'exemple 3 est de 6,2.

Ceci démontre l'excellente sélectivité des produits de l'invention.

### ◆ Test de l'hypothermie chez le cobaye

Les cobayes sont stockés en batterie par trois, avec libre accès à la nourriture et à l'eau, pendant une semaine avant d'entrer dans l'étude. Une heure avant chaque expérience, les cobayes sont placés dans des cages individuelles avec libre accès à l'eau. Ils sont remis dans leur batterie respective à la fin de chaque expérience. Les mesures de température sont effectuées à l'aide d'un thermomètre digital et d'une sonde rectale. Chaque cobaye est pesé, on prend sa température corporelle basale puis on injecte i.p. ou p.o. le composé de la présente invention à évaluer.
Dans le cas d'un agoniste, on prend la température corporelle de chaque cobaye toutes les 30 minutes pendant 2 heures.
Dans le cas d'un antagoniste, 15 minutes après son injection, on injecte à nouveau chaque cobaye i.p. avec l'agoniste prototypique 5HT₁B : GR46611 (5mg/kg). On prend alors la température pendant 2 heures toutes les 30 minutes.
Le critère de jugement utilisé est la différence de température à un temps donné par rapport à la température basale. Pour chaque dose de produit et pour chaque temps (t₃₀, t₆₀, t₉₀, t₁₂₀) on calcule la moyenne et l'erreur standard à la moyenne.
A titre d'exemple et pour illustrer les effets des produits de l'invention à t₉₀ et par voie ip, on rapporte dans le tableau suivant les résultats du composé de l'exemple 1 qui se comporte comme un antagoniste.

| **Injection 1 (a)** | **Injection 2 (a)** | **ΔT °C** **(à 90 minutes) (b)** |
|---|---|---|
| Véhicule | Véhicule | 0 ± 0,1 |
| Véhicule | GR 46611 5 mg/kg | - 1,04 ± 0,15 |
| Produit de l'exemple 1 0,04 mg/kg | GR 46611 5 mg/kg | - 0,82 ± 0,30 |
| Produit de l'exemple 1 0,16 mg/kg | GR 46611 5 mg/kg | - 0,75 ± 0,22 |
| Produit de l'exemple 1 0,63 mg/kg | GR 46611 5 mg/kg | - 0,15∗ ± 0,10 |

| | | |
|---|---|---|
| (a) : voie d'administration i.p. | | |
| (b) : les valeurs sont les moyennes ± S.E.M N≥6 par valeur | | |
| ∗ : p < 0,05 versus véhicule / GR 46611 selon le test de Dunnett | | |

### ◆ Expérience de microdialyse chez le rat

Les rats sont anesthésiés au pentobarbital (60mg/kg i.p.). Ils sont placés dans un appareil stéréotaxique de Kopf et le guide de la canule est implanté dans le cortex frontal cingulé suivant les coordonnées décrites comme suit dans l'atlas de Paxinos et Watson (1982) : AP : + - 2.2, L : ± 0.6, DV : - 0.2. Les rats sont mis en cage séparément et ne sont utilisés en dialyse que 5 jours plus tard. Le jour de la dialyse la sonde est descendue lentement et maintenue dans sa position. La sonde est perfusée à un débit de 1 µl/mn avec une solution de 147.2 mM de NaCl, 4 mM de KCI et 2,3 mM de CaCl₂ amené à pH 7,3 avec un tampon phosphate (0,1 M). Deux heures après l'implantation, les échantillons sont collectés toutes les 20 minutes pendant 4 heures. Trois échantillons de base sont collectés avant l'administration des produits à tester. Les rats sont laissés dans leur cage individuelle pendant toute l'expérience. A la fin de l'expérience, les rats sont décapités et le cerveau prélevé est congelé dans l'isopentane. Des sections d'une épaisseur de 100 µm sont coupées et colorées avec du cresyl violet, ce qui permet la vérification de l'emplacement des sondes.
La quantification simultanée de dopamine, norépinéphrine et sérotonine est effectuée de la façon suivante : 20 µl d'échantillons de dialyse sont dilués avec 20 µl de phase mobile (NaH₂PO₄ : 75 mM, EDTA : 20 µM, sodium 1-decanesulfonate : 1 mM, méthanol : 17,5%, triethylamine : 0,01 %, pH : 5,70) et 33 µl sont analysés par HPLC avec une colonne en phase inverse (hypersil ODS 5 µm, C18, 150 x 4,6 mm, Thermo Separation Products, Les Ulis, France) thermostatée à 45° C et quantifiés par l'intermédiaire d'un détecteur coulométrique. Le potentiel de la première électrode du détecteur est fixée à - 90 mV (réduction) et la seconde à + 280 mV (oxydation). La phase mobile est injectée avec une pompe Beckman 116 à un débit de 2ml/mn. Les limites de sensibilité pour la dopamine, la norépinéphrine et la sérotonine sont de 0.55 fmole par échantillon. Tous les produits de l'invention sont injectés par voie sous cutanée dans un volume de 1,0 ml/kg. Les produits sont dissous dans de l'eau distillée additionnée de quelques gouttes d'acide lactique si nécessaire.
Dans le cas des agonistes on a observé une diminution de la concentration extracellulaire de sérotonine (voir tableau suivant).
Dans le cas des antagonistes on a observé la réversion de la diminution de la concentration extracellulaire de sérotonine produite par l'agoniste GR46611 injecté 20 minutes après les composés de l'invention à tester (voir tableau suivant).

| ***Véhicule* + *Véhicule*** | ***Véhicule* + *GR 46,611 (10.0 mg*/*kg, s.c.)*** | ***Produit de l'exemple 1 (10.0 mg*/*kg, s.c.)* + *Vehicule*** | ***Produit de l'exemple 1 (10.0 mg*/*kg, s.c.)* + *GR 46,611 (10.0 mg*/*kg,s.c.)*** |
|---|---|---|---|
| M ± E.S.M (n) | M ± E.S.M (n) | M ± E.S.M (n) | M ± E.S.M (n) |
| + 2,0 ± 2,5 (10) | - 40,7 ± 2,3 (5) | - 9,5 ± 3,7 (8) | + 0,7 ± 3,4 (6) |

M ± E.S.M = Moyenne de l'effet tous temps confondus après administration des produits ± Erreur Standard à la Moyenne et n = nombre d'animaux. Les quantités de neurotransmetteurs sont exprimées comme une fonction de la moyenne des trois valeurs de base, avant administration des produits et définies comme 0 %. Le niveau basal correspond à 0,59 ± 0,08 pg/20 µl de microdialysat, N = 10. La réversion de la diminution de la concentration extracellulaire de sérotonine produite par le GR 46,611 est évaluée statistiquement par une analyse de variance avec les produits comme facteur contrôlés, F (1,25) = 26,5, P < 0,01.

## Revendications

1. Les composés de 2-amino indane de formule I : 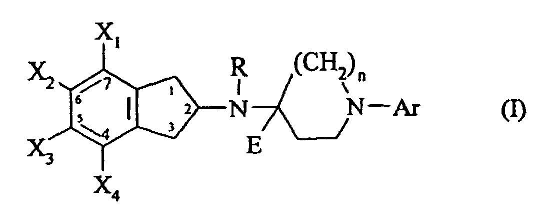 dans laquelle :
- n représente 2 ;
- Ar représente : 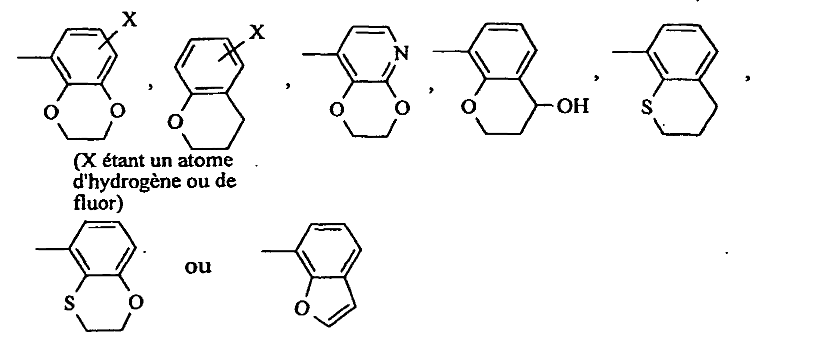
- R représente un atome d'hydrogène,
- E représente un atome d'hydrogène ou un radical méthyle, et
- X₁, X₂, X₃ et X₄, identiques ou différents représentent
. chacun un atome d'hydrogène ou d'halogène, un radical (C₁-C₅)alkyle ou (C₁-C₅)alkoxy chacun en chaîne droite ou ramifiée, un radical trifluorométhyle, hydroxy, nitro, ou 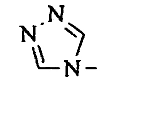
. et/ou pris deux à deux en position adjacente forment, avec les atomes de carbone du noyau phényle auquel ils sont liés un cycle pentagonal composé d'atomes choisis parmi les atomes de carbone, oxygène et azote,
sous forme de mélange racémique et d'isomères optiques quand ils existent,
et leurs sels d'addition acides physiologiquement tolérables.

2. Un composé de la revendication 1 choisi parmi le groupe constitué par les :
1-(2,3-dihydro[1,4] benzodioxin-5-yl)-4-[N-(indan-2-yl) amino] pipéridine et son dichlorhydrate
1-(2,3-dihydro[1,4] benzodioxin-5-yl)-4-[N-(5,6-méthylènedioxy indan-2-yl) amino] pipéridine et son fumarate
4-[N-(indan-2-yl) amino]-1-(thiochroman-8-yl) pipéridine
1-(2,3-dihydro[1,4] benzodioxin-5-yl)-4-[N-(5,6-diméthoxy indan-2-yl) amino] pipéridine et son fumarate
1-(2,3-dihydro[1,4] benzodioxin-5-yl)-4-[N-(5-méthyl indan-2-yl) amino] pipéridine et son fumarate
1-(2,3-dihydro[1,4] benzodioxin-5-yl)-4-[N-(5-chloro indan-2-yl) amino] pipéridine et son fumarate
1-(2,3-dihydro [1,4] benzodioxin-5-yl)-4-[N-(5-méthoxy indan-2-yl)amino] pipéridine et son fumarate
1-(2,3-dihydro [1,4] benzodioxin-5-yl)-4-[N-(5,6-diméthyl indan-2-yl) amino] pipéridine et son fumarate
1-(2,3-dihydro[1,4]benzodioxin-5-yl)-4-[N-(5-trifluorométhyl indan-2-yl) amino] pipéridine et son fumarate
1-(2,3-dihydro [1,4] benzodioxin-5-yl)-4-[N-(4,7-diméthoxy indan-2-yl) amino]pipéridine et son hémifumarate
1-(2,3-dihydro [1,4] benzodioxin-5-yl)-4-[N-(5-fluoroindan-2-yl)amino] pipéridine et son fumarate
1-(2,3-dihydro [1,4] benzoxathiin-5-yl)-4-(indan-2-yl amino) pipéridine et son hémifumarate
1-(chroman-8-yl)-4-(indan-2-yl amino)pipéridine et son fumarate
1-(6-fluoro chroman-8-yl)-4-(indan-2-yl amino) pipéridine et son fumarate
1-(2,3-dihydro [1,4] benzodioxin-5-yl)-4-(5-nitro indan-2-yl amino) pipéridine et son fumarate
1-(chroman-8-yl)-4-[5-(1,2,4-triazol-4-yl) indan-2-yl amino] pipéridine et son hémifumarate
1-(chroman-8-yl)-4-[(cyclopenta [f] [2,1,3] benzoxadiazol-6-yl) amino] pipéridine et son fumarate
1-(chroman-8-yl)-4-[(5-fluoro indan-2-yl)amino]pipéridine et son fumarate
1-(2,3-dihydrobenzo furan-7-yl)-4-[(5-fluoro indan-2-yl)amino] pipéridine et son hémifumarate 1-(benzofuran-7-yl)-4-[(5-fluoro indan-2-yl)amino] pipéridine et son hémifumarate
1-(chroman-8-yl)-4-[(5-méthoxyindan-2-yl)amino]pipéridine et son fumarate
1-(4-hydroxy chroman-8-yl)-4-[(5,6-méthylènedioxy indan-2-yl)amino] pipéridine et son hémifumarate
1-(2,3-dihydro[1,4] benzodioxin-5-yl)-4-(indan-2-yl amino)-4-méthyl pipéridine et son fumarate
1-(2,3-dihydro[1,4]-benzodioxin-5-yl)-4-[N-(5-hydroxy indan-2-yl)amino]pipéridine et son hémifumarate
1-(chroman-8-yl)-4-[N-(5-hydroxy indan-2-yl)amino]pipéridine et son hémifumarate

3. Un composé selon les revendications 1 et 2 qui est la 1-(2,3-dihydro[1,4] benzodioxin-5-yl)-4-[N-(indan-2-yl) amino] pipéridine et son dichlorhydrate

4. Un composé selon les revendications 1 et 2 qui est la 1-(2,3-dihydro[1,4] benzodioxin-5-yl)-4-[N-(5,6-méthylènedioxy indan-2-yl) amino] pipéridine et son fumarate

5. Un composé selon les revendications 1 et 2 qui est la 1-(2,3-dihydro [1,4] benzodioxin-5-yl)-4-[N-(5-fluoroindan-2-yl)amino] pipéridine et son fumarate

6. Un composé selone les revendications 1 et 2 qui est la 1-(chroman-8-yl)-4-(indan-2-yl amino)pipéridine et son fumarate

7. Un composé selon les revendications 1 et 2 qui est la 1-(2,3-dihydro[1,4]-benzodioxin-5-yl)-4-[N-(5-hydroxy indan-2-yl)amino]pipéridine et son hémifumarate

8. Le procédé de préparation des composés de la revendication 1 **caractérisé en ce que** l'on fait réagir :
- soit un composé de formule II : 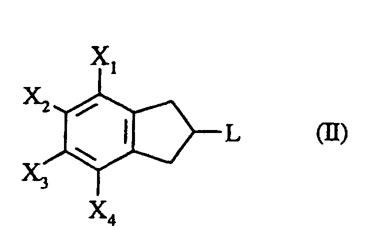
dans laquelle X₁, X₂, X₃ et X₄ ont les significations définies dans la revendication 1 et L est
un groupement labile,
sur un composé de formule III : 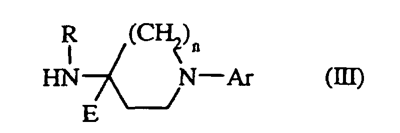 dans laquelle R, E, n et Ar ont les significations définies dans la revendication 1,
- soit un composé de formule IV : 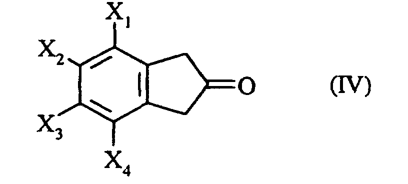
dans laquelle X₁, X₂, X₃ et X₄ ont les significations précédemment définies,
sur un composé de formule III précédemment définie,
au cours d'une réaction d'amination réductrice, en présence de borohydrure de sodium dans le tétrahydrofurane ou de triacétoxylborohydrure de sodium dans le dichloroéthane,
- soit encore lorsque E représente uniquement un atome d'hydrogène, un composé de formule V : 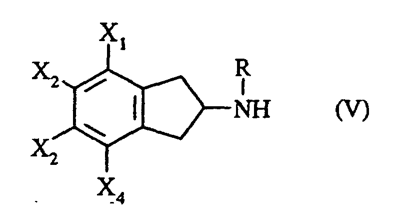 dans laquelle X₁, X₂, X₃, X₄ et R ont les significations précédemment définies,
sur un composé de formule VI : 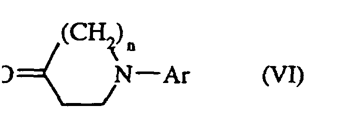 dans laquelle n et Ar ont les significations précédemment définies,
au cours d'une réaction d'amination réductrice, en présence de borohydrure de sodium dans le tétrahydrofurane ou de triacétoxyborohydrure de sodium dans le dichloroéthane, pour obtenir un composé de formule I' : 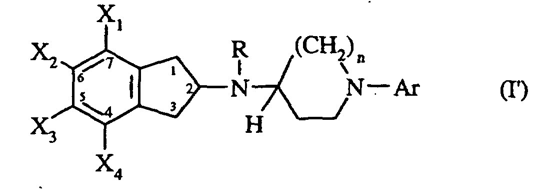 dans laquelle X₁, X₂, X₃, X₄, R, Ar et n ont les significations précédemment définies, (l'étant un sous-ensemble de I)
- soit enfin lorsque le groupement Ar prend la valeur 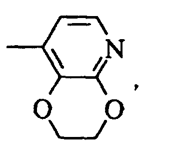 un composé de formule VII : 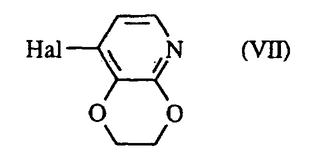 dans laquelle Hal représente un atome d'halogène choisi parmi les atomes de chlore, brome et iode,
sur un composé de formule VIII : 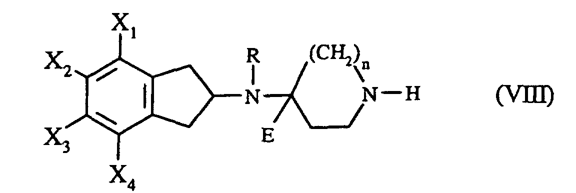 dans laquelle X₁, X₂, X₃, X₄, R, E et n ont les significations précédemment définies,
en opérant à chaud, dans un solvant approprié, pour obtenir un composé de formule I" : 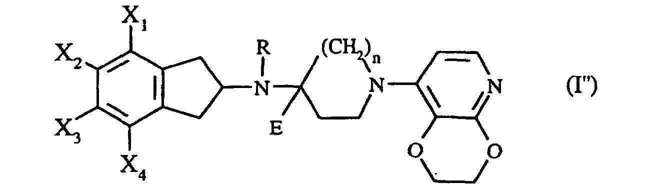 dans laquelle X₁, X₂, X₃, X₄, R, E et n ont les significations précédemment définies,
(I" étant un autre sous-ensemble de I) ;
et lorsque l'un ou plusieurs des substituants X₁, X₂, X₃ et X₄ prennent la valeur hydroxy, on prépare les composés de formule I précédemment définie et dans laquelle un ou plusieurs des substituants X₁, X₂, X₃ et X₄ représentent un radical hydroxy, à partir des dérivés méthoxy correspondants que l'on traite par le chlorhydrate de pyridine à 200 °C,
en plus, si on le désire, on prépare les isomères des composés de formule I lorsqu'ils renferment un ou plusieurs carbones asymétriques, selon les méthodes classiques de dédoublement, ou à partir de matières premières optiquement actives,
et le cas échéant, les composés I ainsi obtenus sont traités avec des acides pharmaceutiquement acceptables pour obtenir les sels d'addition acides correspondants.

9. Les compositions pharmaceutiques utilisables dans le traitement de la dépression, l'anxiété, les troubles impulsifs, l'obésité et les maladies psychiatriques liées à un dysfonctionnement de la transmission sérotoninergique, contenant comme principe actif un composé selon une des revendications 1 à 6, avec un ou plusieurs excipients pharmaceutiquement appropriés.

## Claims

1. 2-Aminoindan compounds of formula I : 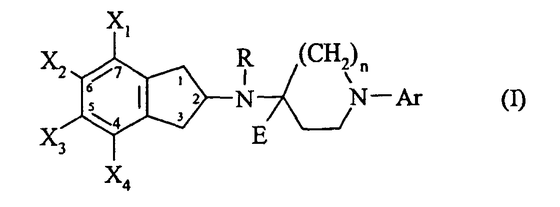 wherein :
- n represents 2 ;
- Ar represents : 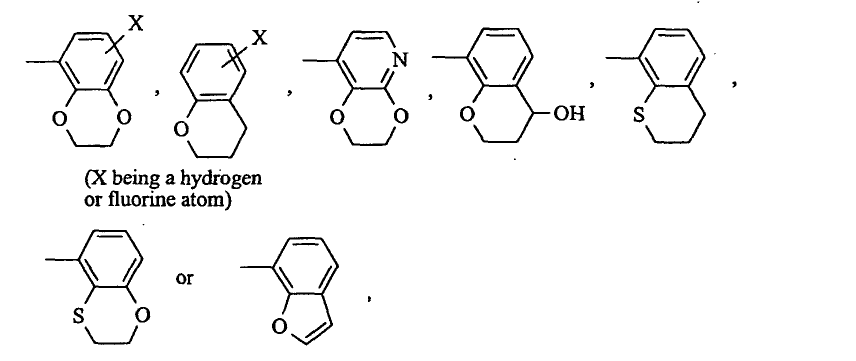
- R represents a hydrogen atom,
- E represents a hydrogen atom or a methyl radical, and
- X₁, X₂, X₃ and X₄, which may be identical or different,
. each represents a hydrogen or halogen atom, a straight-chain or branched (C₁-C₅)-alkyl or (C₁-C₅)-alkoxy radical, or a trifluoromethyl, hydroxy, nitro or 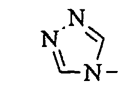 radical,
. and/or a pair adjacent to one another form, together with the carbon atoms of the phenyl nucleus to which they are bonded, a 5-membered ring composed of atoms selected from the atoms carbon, oxygen and nitrogen,
where they exist in the form of a racemic mixture or in the form of optical isomers,
and the physiologically tolerable acid addition salts thereof.

2. A compound of claim 1 selected from the group consisting of :
1-(2,3-dihydro[1,4]benzodioxin-5-yl)-4-[N-(indan-2-yl)amino]piperidine and its dihydrochloride
1-(2,3-dihydro[ 1,4]benzodioxin-5-yl)-4-[N-(5,6-methylenedioxyindan-2-yl)amino]-piperidine and its fumarate
4-[N-(indan-2-yl)amino]-1-(thiochroman-8-yl)piperidine
1-(2,3-dihydro[1,4]benzodioxin-5-yl)-4-[N-(5,6-dimethoxyindan-2-yl)amino]piperidine and its fumarate
1-(2,3-dihydro[1,4]benzodioxin-5-yl)-4-[N-(5-methylindan-2-yl)amino]piperidine and its fumarate
1-(2,3-dihydro[1,4]benzodioxin-5-yl)-4-[N-(5-chloroindan-2-yl)amino]piperidine and its fumarate
1-(2,3-dihydro[1,4]benzodioxin-5-yl)-4-[N-(5-methoxyindan-2-yl)amino]piperidine and its fumarate
1-(2,3-dihydro[1,4]benzodioxin-5-yl)-4-[N-(5,6-dimethylindan-2-yl)amino]piperidine and its fumarate
1-(2,3-dihydro[1,4]benzodioxin-5-yl)-4-[N-(5-trifluoromethylindan-2-yl)-amino]piperidine and its fumarate
1-(2,3-dihydro[1,4]benzodioxin-5-yl)-4-[N-(4,7-dimethoxyindan-2-yl)amino]piperidine and its hemifumarate
1-(2,3-dihydro[1,4]benzodioxin-5-yl)-4-[N-(5-fluoroindan-2-yl)amino]piperidine and its fumarate
1-(2,3-dihydro[1,4]benzoxathiin-5-yl)-4-(indan-2-ylamino)piperidine and its hemifumarate
1-(chroman-8-yl)-4-(indan-2-ylamino)piperidine and its fumarate
1-(6-fluorochroman-8-yl)-4-(indan-2-ylamino)piperidine and its fumarate
1-(2,3-dihydro[1,4]benzodioxin-5-yl)-4-(5-nitroindan-2-ylamino)piperidine and its fumarate
1-(chroman-8-yl)-4-[5-(1,2,4-triazol-4-yl)indan-2-ylamino]piperidine and its hemifumarate
1-(chroman-8-yl)-4-[(cyclopenta[f][2,1,3]benzoxadiazol-6-yl)amino]piperidine and its fumarate
1-(chroman-8-yl)-4-[(5-fluoroindan-2-yl)amino]piperidine and its fumarate
1-(2,3-dihydrobenzofuran-7-yl)-4-[(5-fluoroindan-2-yl)amino]piperidine and its hemifumarate
1-(benzofuran-7-yl)-4-[(5-fluoroindan-2-yl)amino]piperidine and its hemifumarate
1-(chroman-8-yl)-4-[(5-methoxyindan-2-yl)amino]piperidine and its fumarate
1-(4-hydroxychroman-8-yl)-4-[(5,6-methylenedioxyindan-2-yl)amino]piperidine and its hemifumarate
1-(2,3-dihydro[1,4]benzodioxin-5-yl)-4-(indan-2-ylamino)-4-methylpiperidine and its fumarate
1-(2,3-dihydro[1,4]-benzodioxin-5-yl)-4-[N-(5-hydroxyindan-2-yl)amino]piperidine and its hemifumarate
1-(chroman-8-yl)-4-[N-(5-hydroxyindan-2-yl)amino]piperidine and its hemifumarate.

3. A compound according to claims 1 and 2 which is 1-(2,3-dihydro[1,4]benzodioxin-5-yl)-4-[N-(indan-2-yl)amino]piperidine or its dihydrochloride.

4. A compound according to claims 1 and 2 which is 1-(2,3-dihydro[1,4]benzodioxin-5-yl)-4-[N-(5,6-methylenedioxyindan-2-yl)amino]piperidine or its fumarate.

5. A compound according to claims 1 and 2 which is 1-(2,3-dihydro[1,4]benzodioxin-5-yl)-4-[N-(5-fluoroindan-2-yl)amino]piperidine or its fumarate.

6. A compound according to claims 1 and 2 which is 1-(chroman-8-yl)-4-(indan-2-ylamino)piperidine or its fumarate.

7. A compound according to claims 1 and 2 which is 1-(2,3-dihydro[1,4]benzodioxin-5-yl)-4-[N-(5-hydroxyindan-2-yl)amino]piperidine or its hemifumarate.

8. A process for the preparation of compounds of claim 1, **characterised in that** :
- a compound of formula II : 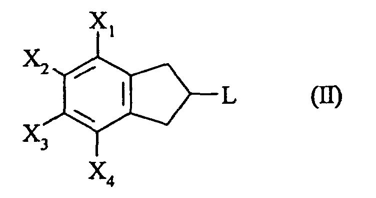
wherein X₁, X₂, X₃ and X₄ are as defined in claim 1 and L is a labile group is reacted with a compound of formula III : 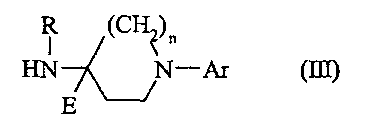 wherein R, E, n and Ar are as defined in claim 1,
or
- a compound of formula IV : 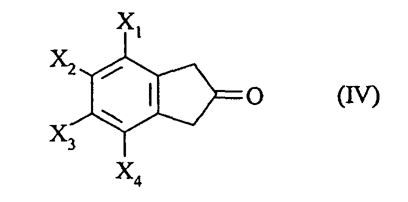
wherein X₁, X₂, X₃ and X₄ are as defined hereinbefore is reacted
with a compound of formula III defined above,
in a reductive amination reaction, in the presence of sodium borohydride in tetrahydrofuran or sodium triacetoxyborohydride in dichloroethane,
or
- when E represents a hydrogen atom only, a compound of formula V : 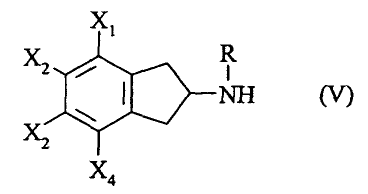
wherein X₁, X₂, X₃, X₄ and R are as defined hereinbefore is reacted
with a compound of formula VI : 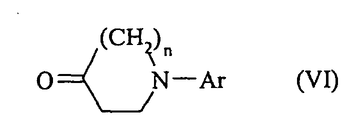 wherein n and Ar are as defined hereinbefore,
in a reductive amination reaction, in the presence of sodium borohydride in tetrahydrofuran or sodium triacetoxyborohydride in dichloroethane,
to obtain a compound of formula I' : 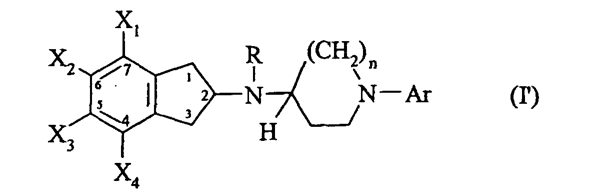 wherein X₁, X₂, X₃, X₄, R, Ar and n are as defined hereinbefore,
(I' being a sub-set of I) ,
or, finally,
- when the group Ar represents 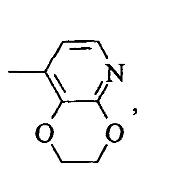 a compound of formula VII : 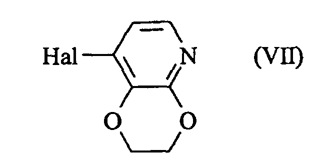
wherein Hal represents a halogen atom selected from the atoms chlorine, bromine and iodine, is reacted
with a compound of formula VIII : 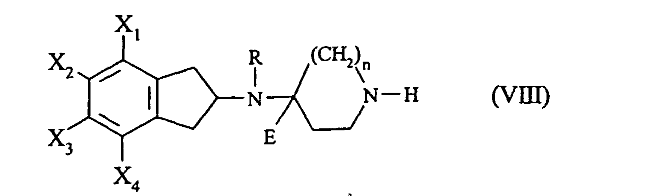 wherein X₁, X₂, X₃, X₄, R, E and n are as defined hereinbefore,
with the application of heat, in an appropriate solvent, to obtain a compound of formula I" : 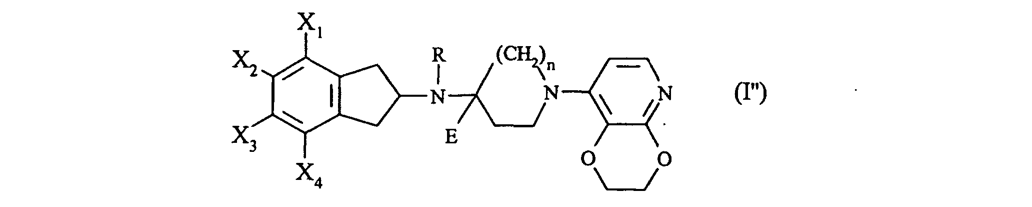 wherein X₁, X₂, X₃, X₄, R, E and n are as defined hereinbefore,
(I" being another sub-set of I) ;
and, when one or more of the substituents X₁, X₂, X₃ and X₄ represent(s) hydroxy, the compounds of formula I defined above in which one or more of the substituents X₁,
X₂, X₃ and X₄ represent(s) a hydroxy radical are prepared from corresponding
methoxy compounds which are treated with pyridine hydrochloride at 200°C,
and furthermore, if desired, when the compounds of formula I contain one or more asymmetric carbon atoms, the isomers thereof are prepared according to conventional methods of separation or starting from optically active starting materials,
and, optionally, the compounds I so obtained are treated with pharmaceutically acceptable acids to obtain the corresponding acid addition salts.

9. Pharmaceutical compositions that can be used in the treatment of depression, anxiety, impulsive disorders, obesity and psychiatric disorders associated with dysfunction of serotoninergic transmission, comprising as active ingredient a compound according to any one of claims 1 to 6, with one or more pharmaceutically appropriate excipients.

## Patentansprüche

1. 2-Amino-indan-Verbindungen der Formel I: 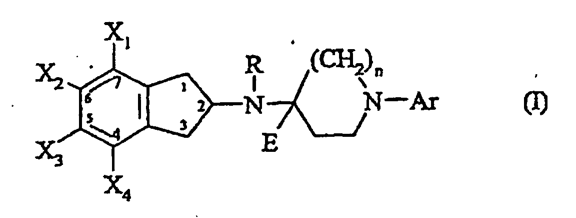 in der:
- n den Wert 2 besitzt;
- Ar: 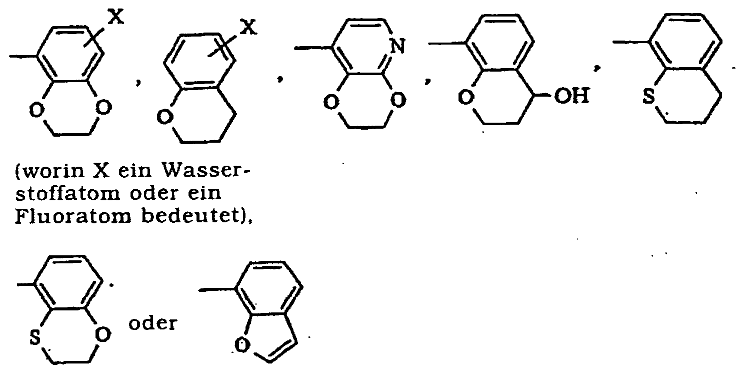 bedeutet;
- R ein Wasserstoffatom bedeutet;
- E ein Wasserstoffatom oder eine Methylgruppe darstellt; und
- X₁, X₂, X₃ und X₄, die gleichartig oder verschieden sind,
. jeweils ein Wasserstoffatom, ein Halogenatom, eine (C₁-C₅)-Alkyl- oder (C₁-C₅)-Alkoxygruppe mit gerader oder verzweigter Kette, eine Trifluormethylgruppe, Hydroxygruppe, Nitrogruppe oder eine Gruppe der Formel 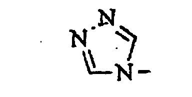 bedeuten
. und/oder jeweils zu zweit in benachbarter Position zusammen mit den Kohlenstoffatomen des Phenylkerns, an den sie gebunden sind, einen fünfeckigen Ring bilden aus Atomen, die aus Kohlenstoff-, Sauerstoff- und Stickstoffatomen ausgewählt sind,
in Form der racemischen Mischung und in Form der optischen Isomeren, falls diese existieren, und deren physiologisch verträgliche Säureadditionssalze.

2. Verbindung nach Anspruch 1, ausgewählt aus der Gruppe, die gebildet wird durch:
1-(2,3-Dihydro[1,4]benzodioxin-5-yl)-4-[N-(indan-2-yl)-amino]-piperidin und dessen Dihydrochlorid,
1-(2,3-Dihydro[1,4]benzodioxin-5-yl)-4-[N-(5,6-methylendioxy-indan-2-yl)-amino]-piperidin und dessen Fumarat,
4-[N-(Indan-2-yl)-amino)-1-(thiochroman-8-yl)-piperidin,
1-(2,3-Dihydro[1,4]benzodioxin-5-yl)-4-[N-(5,6-dimethoxy-indan-2-yl)-amino]-piperidin und dessen Fumarat,
1-(2,3-Dihydro[1,4]benzodioxin-5-yl)-4-[N-(5-methyl-indan-2-yl)-amino]-piperidin und dessen Fumarat,
1-(2,3-Dihydro[1,4]benzodioxin-5-yl)-4-[N-(5-chlor-indan-2-yl)-amino]-piperidin und dessen Fumarat,
1-(2,3-Dihydro[1,4]benzodioxin-5-yl)-4-[N-(5-methoxy-indan-2-yl)-amino]-pipertdin und dessen Fumarat,
1-(2,3-Dihydro[ 1,4]benzodioxin-5-yl)-4-[N-(5,6-dimethyl-indan-2-yl)-amino]-piperidin und dessen Fumarat,
1-(2,3-Dihydro[ 1,4]benzodioxin-5-yl)-4-[N-(5-trifluormethyl-indan-2-yl)-amino]-piperidin und dessen Fumarat,
1-(2,3-Dihydro[1,4]benzodioxin-5-yl)-4-[N-(4,7-dimethoxy-indan-2-yl)-amino]-piperidin und dessen Hemifumarat,
1-(2,3-Dihydro[1,4]benzodioxin-5-yl)-4-(N-(5-fluorindan-2-yl)-amino]-piperidin und dessen Fumarat,
1-(2,3-Dihydro[1,4]benzoxathiin-5-yl)-4-(indan-2-yl-amino)-piperidin und dessen Hemifumarat.
1-(Chroman-8-yl)-4-(indan-2-yl-amino)-piperidin und dessen Fumarat,
1-(6-Fluor-chroman-8-yl)-4-(indan-2-yl-amino)-piperidin und dessen Fumarat,
1-(2,3-Dihydro[1,4]benzodioxin-5-yl)-4-(5-nitro-indan-2-yl-amino)-piperidin und dessen Fumarat,
1-(Chroman-8-yl)-4-[5-(1,2,4-triazol-4-yl)-indan-2-yl-amino)-piperidin und dessen Hemifumarat,
1-(Chroman-8-yl)-4-[(cyclopenta[f][2,1,3]benzoxadiazol-6-yl)-amino]-piperidin und dessen Fumarat,
1-(Chroman-8-yl)-4-[(5-fluorindan-2-yl)-aminol-piperidin und dessen Fumarat,
1-(2,3-Dihydrobenzo-furan-7-yl)-4-[(5-fluorindan-2-yl)-amino]-piperidin und dessen Hemifumarat.
1-(Benzofuran-7-yl)-4-[(5-fluorindan-2-yl)-amino]-piperidin und dessen Hemifumarat,
1-(Chroman-8-yl)-4-[(5-methoxyindan-2-yl)-amino]-piperidin und dessen Fumarat,
1-(4-Hydroxychroman-8-yl)-4-[(5,6-methylendioxy-indan-2-yl)-amino]-piperidin und dessen Hemifumarat,
1-(2,3-Dihydro[1,4]benzodioxin-5-yl)-4-(indan-2-yl-amino)-piperidin und dessen Fumarat,
1-(2,3-Dihydro[1,4]benzodioxin-5-yl)-4-[N-(5-hydroxy-indan-2-yl)-amino]-piperidin und dessen Hemifumarat,
1-(Chroman-8-yl)-4-[N-(5-hydroxy-indan-2-yl)-amino]-piperidin und dessen Hemifumarat.

3. Verbindung nach einem der Ansprüche 1 und 2, nämlich 1-(2,3-Dihydro[1,4]benzodioxin-5-yl)-4-[N-(indan-2-yl)-amino]-piperidin und dessen Dihydrochlorid.

4. Verbindung nach einem der Ansprüche 1 und 2, nämlich 1-(2,3-Dihydro[ 1,4]benzodioxin-5-yl)-4-[N-(5,6-methylendioxy-indan-2-yl)-amino]-piperidin und dessen Fumarat.

5. Verbindung nach einem der Ansprüche 1 und 2, nämlich 1-(2,3-Dihydro[1,4]benzodioxin-5-yl)-4-[N-(5-fluorindan-2-yl)-amino]-piperidin und dessen Fumarat.

6. Verbindung nach einem der Ansprüche 1 und 2, nämlich 1-(Chroman-8-yl)-4-(indan-2-yl)-amino)-piperidin und dessen Fumarat.

7. Verbindung nach einem der Ansprüche 1 und 2, nämlich 1-(2,3-Dihydro[1,4]benzodioxin-5-yl)-4-[N-(5-hydroxy-indan-2-yl)-amino]-piperidin und dessen Hemifumarat.

8. Verfahren zur Herstellung der Verbindungen nach Anspruch 1, **dadurch gekennzeichnet, daß** man:
- entweder eine Verbindung der Formel II: 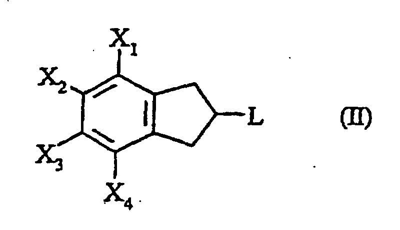 in der X₁, X₂, X₃ und X₄ die in Anspruch 1 angegebenen Bedeutungen besitzen und L für eine labile Gruppe steht.
mit einer Verbindung der Formel III: 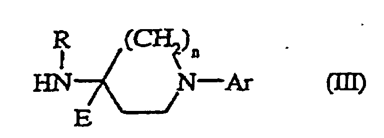 in der R, E; n und Ar die in Anspruch 1 angegebenen Bedeutungen besitzen, umsetzt,
- oder eine Verbindung der Formel IV: 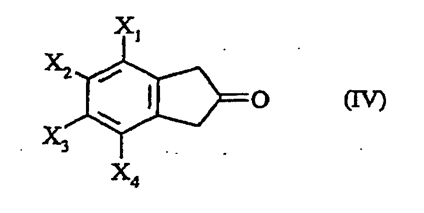 in der X₁, X₂, X₃ und X₄ die oben angegebenen Bedeutungen besitzen, mit einer Verbindung der Formel III, wie sie oben definiert worden ist, im Verlaufe einer reduzierenden Aminierungsreaktion in Gegenwart von Natriumborhydrid in Tetrahydrofuran oder Natriumtriacetoxyborhydrid in Dichlorethan umsetzt,
- oder, wenn E ausschließlich ein Wasserstoffatom bedeutet, eine Verbindung der Formel V: 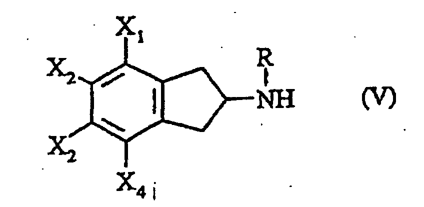 in der X₁, X₂, X₃, X₄ und R die oben angegebenen Bedeutungen besitzen, mit einer Verbindung der Formel VI: 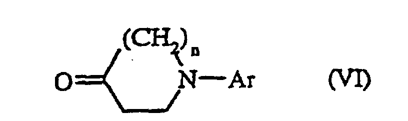 in der n und Ar die oben angegebenen Bedeutungen besitzen, im Verlaufe einer reduzierenden Aminierungsreaktion in Gegenwart von Natriumborhydrid in Tetrahydrofuran oder von Natriumtriacetoxyborhydrid in Dichlorethan umsetzt zur Bildung einer Verbindung der Formel I': 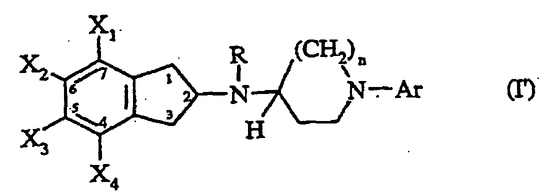 in der X₁, X₂, X₃, X₄, R, Ar und n die oben angegebenen Bedeutungen besitzen,
(wobei I' eine Untergruppe von I darstellt)
- oder wenn die Gruppe Ar die Bedeutung 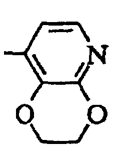 annimmt,
eine Verbindung der Formel VII: 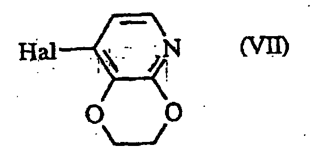 in der Hal ein Halogenatom, ausgewählt aus Chlor-, Brom- und Iodatomen darstellt,
mit einer Verbindung der Formel VIII: 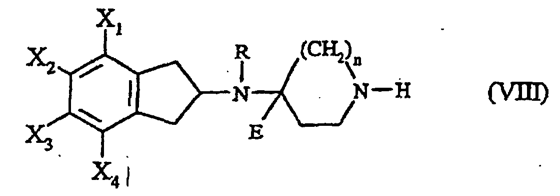 in der X₁, X₂, X₃, X₄, R, E und n die oben angegebenen Bedeutungen besitzen, umsetzt,
wobei man in der Wärme in einem geeigneten Lösungsmittel arbeitet, zur Bildung einer Verbindung der Formel I": 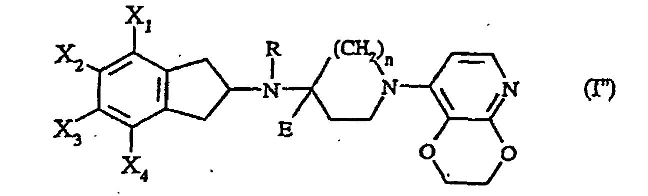 in der X₁, X₂, X₃, X₄, R, E und n die oben angegebenen Bedeutungen besitzen,
(wobei I" eine andere Untergruppe von I darstellt);
und wenn einer oder mehrere der Substituenten X₁, X₂, X₃ und X₄ eine Hydroxygruppe bedeutet, man die Verbindungen der oben definierten Formel I, in der einer oder mehrere der Substituenten X₁, X₂, X₃ und X₄ eine Hydroxygruppe darstellt, ausgehend von den Methoxy-Derivaten, die man mit Pyridin-Hydrochlorid bei 200°C behandelt, herstellt,
und man gewünschtenfalls die Isomeren der Verbindungen der Formel I, wenn diese ein oder mehrere asymmetrische Kohlenstoffatome enthalten, mit Hilfe klassischer Aufspaltungsmethoden oder ausgehend von optisch aktiven Ausgangsmaterialien herstellt,
und man gegebenenfalls die in dieser Weise erhaltenen Verbindungen mit pharmazeutisch annehmbaren Säuren behandelt zur Bildung der entsprechenden Säureadditionssalze.

9. Pharmazeutische Zubereitungen für die Behandlung der Depression, der Angst, von Impulsivitätsstörungen, der Fettsucht und psychiatrischen Erkrankungen, die mit einer Dysfunktion der serotoninergischen Transmission verknüpft sind, enthaltend als Wirkstoff eine Verbindung nach einem der Ansprüche 1 bis 6 zusammen mit einem oder mehreren pharmazeutisch geeigneten Trägermaterialien.
